# EUROPEAN PATENT APPLICATION

(11) **EP 4 491 190 A1**
(43) Date of publication of application: **15.01.2025**
(21) Application number: 23382726.0
(22) Date of filing: 14.07.2023
(51) Int. Cl.: A61K 38/17, A61P 3/00, A61P 3/04, A61P 3/06, A61P 17/00, A61P 19/00, A61P 29/00, A61P 35/00, C07K 14/435

(54) **SMALL PROTEINS FOR USE AS SENOLYTIC AND SENOMORPHIC AGENTS**

(71) Applicant: Fundación Imdea Alimentación, 28049 Madrid (ES); Fundació Institut de Recerca Biomèdica (IRB Barcelona), 08028 Barcelona (ES); Institució Catalana de Recerca I Estudis Avançats, 08010 Barcelona (ES)
(72) Inventor: IKONOMOPOULOU, Maria, 28049 MADRID (ES); MORAL SANZ, Javier, 28049 MADRID (ES); SERRANO, Manuel, 08028 BARCELONA (ES); RAMPONI, Valentina, 08028 BARCELONA (ES); FERNANDEZ ROJO, Manuel A., 28049 MADRID (ES); FERNANDEZ CARRASCO, Isabel, 28049 MADRID (ES)
(74) Representative: Herrero & Asociados, S.L.

(57) **Abstract**

The present invention refers to venom-derived small proteins from a marine source for use as a senolytic and/or senomorphic agent in the prevention and treatment of aging and age-related diseases. The invention also refers to pharmaceutical and cosmetic compositions comprising these proteins. Finally, the invention refers to a method for identifying compounds effective for the treatment or prevention of aging and age-related diseases.

## Description

### FIELD OF THE INVENTION

The present invention refers to the medical field. In particular, it refers to venom-derived proteins from a marine source with senolytic and senomorphic properties and its use in the prevention and treatment of aging and age-related diseases.

### BACKGROUND OF THE INVENTION

Accumulation of senescent cells (SnCs) with age is involved in the progression of different age-related diseases. Senescent cells cease dividing but are metabolic active cells, secreting factors refereed as senescence-associated secretory phenotype (SASP), which involves the secretion of various factors that contribute to chronic inflammation and age-related diseases *(*Chaib, S. et al. Cellular senescence and senolytics: The path to the clinic. Nature Medicine (2022), 28(8), Article 8*;* Gorgoulis, V., et al. Cellular Senescence: Defining a Path Forward. Cell. (2019) 179(4), 813-827*).* The SASP includes inflammatory and pro-apoptotic cytokines (e.g., TNFα, IL-6, IL-8), chemokines, matrix metalloproteinases, TGFβ family members, activins and inhibins, hemostatic and growth factors, bioactive lipids, and exosomes *(Gorgoulis et al. 2019).* These contribute to inflammation and tissue dysfunction and to transfer cytotoxic and senescence-inducing cargos locally and systemically.

As a result, different strategies have emerged lately in order to therapeutically target SnCs. The therapeutic approach that takes advantage of compounds that target SnCs (senotherapeutics) is commonly described as senotherapy. Senotherapeutic agents comprise senolytics (selectively kill SnCs) and senomorphics/senostatics (compounds that modulate SASP).

Senolytic and senomorphic compounds show promising results in aging and age-related diseases, including cancer treatments. In the latter case, senolytics selectively target and eliminate senescent cancer cells, notorious for being resistant to chemotherapy and radiation, with the goal of improving healthspan and delaying or preventing age-related diseases and the accompanied ill phenotypes *(Chaib et al, 2022;* Kirkland, J. L. et al. Senolytic drugs: From discovery to translation. Journal of Internal Medicine. (2020). 288(5), 518-536*;* Muñoz-Espin, D. et al. A versatile drug delivery system targeting senescent cells. EMBO Molecular Medicine. (2018). 10(9), e9355*)*. These compounds, selectively induce apoptosis in senescent cancer cells, with lesser to none effect in non-transformed cells. Therefore, various strategies target senescent cells, leading to cell death, senescence prevention or reversal.

There are several senolytics in preclinical stages, but only a handful of them have reached clinical trials. The following list refers to the most investigated senolytics:
- BH3 mimetics. ABT-263 (navitoclax), ABT-737, A-1331852, A-1155463. BH3 mimetics are small molecules that mimic BH3-proteins by inhibiting the anti-apoptotic BCL-2, BCL-XL and BCL-W proteins *(*Tse, C., et al. ABT-263: A potent and orally bioavailable Bcl-2 family inhibitor. Cancer Research, (2008), 68(9), 3421-3428*).* To date, more than 20 compounds have been identified, and their effects in cancer therapy have been analysed. Among those, navitoclax (N) has been the leading compound. Navitoclax has been validated in a variety of preclinical models showing high potency in killing senescent cells *(*Chang, J., et al. Clearance of senescent cells by ABT263 rejuvenates aged hematopoietic stem cells in mice. Nature Medicine. (2016), 22(1), 78-83*;* Gonzalez-Gualda, E. et al. Galacto-conjugation of Navitoclax as an efficient strategy to increase senolytic specificity and reduce platelet toxicity. Aging Cell, (2020), 19(4), e 13142*;* Sierra-Ramirez, A. et al. Transient metabolic improvement in obese mice treated with navitoclax or dasatinib/quercetin. Aging (Albany NY), (2020), 12(12), 11337-11348). However, it also has significant off-target haematological toxicity, including thrombocytopenia. This narrows its therapeutic window and can preclude its clinical application.
- Dasatinib, quercetin and fisetin. Common senolytic approaches involve the use of flavonoids alone (quercertin, Q or fisetin, F) or combined with dasatinib (D, SRC tyrosine kinase inhibitor, D+Q, D+F). Indeed, these are under clinical trial to test their senolytic efficacy in pulmonary fibrosis, chronic kidney disease and Alzheimer's disease *(Chaib et al., 2022).* However, the poor bioavailability of the flavonoids requires high doses to be effective. Additionally, its efficacy has been questioned in certain models of cellular senescence (e.g., senescent Huh7 cells, chemotherapy-induced senescence in liver cancer, and liver fibrosis) or their capacity to reduce fibrosis (e.g., mouse models of pulmonary fibrosis) (Badaro-Garcia, S., et al. Standard of care drugs do not modulate activity of senescent primary human lung fibroblasts. Scientific Reports, (2023), 13(1), 3654*;* Kovacovicova, K. et al Senolytic Cocktail Dasatinib+Quercetin (D+Q) Does Not Enhance the Efficacy of Senescence-Inducing Chemotherapy in Liver Cancer. Frontiers in Oncology (2018), 8, 459*;* Nambiar, A. et al. Senolytics dasatinib and quercetin in idiopathic pulmonary fibrosis: Results of a phase I, single-blind, single-center, randomized, placebo-controlled pilot trial on feasibility and tolerability. EBioMedicine, (2023), 90, 104481*).*
- Cardiac Glycosides (CGs) is a large family of compounds that target the Na⁺/K⁺ATPase pump. These are represented by Digoxin, Ouabain, Bufalin and Proscillaridin A. Recent studies have shown that these drugs are senolytics, selectively eliminating various senescent cells in vitro and in vivo *(*Guerrero, A. et al. Cardiac glycosides are broad-spectrum senolytics. Nat Metab, (2019), 1(11), 1074-1088*;* Triana-Martinez, F. et al. Identification and characterization of Cardiac Glycosides as senolytic compounds. Nat Commun, (2019), 10(1), 4731*).* Despite the promising preclinical results, there are also some potential disadvantages of using cardiac glycosides as senolytics. One of the main concerns is the risk of toxicity, as these drugs have a narrow therapeutic window and can cause serious side effects such as cardiac arrhythmias and cardiac arrest at high doses *(*Botelho, A. F. M. et al. A review of cardiac glycosides: Structure, toxicokinetics, clinical signs, diagnosis and antineoplastic potential. Toxicon, (2019), 158, 63-68*).*
- Other less studied strategies involve the inhibition of HSP90 (Geldanamycin, Tanespimycin, Alvespimycin), inhibition of FOXO4 with indirect activation of p53 (FOXO4-related peptide) and MDM2 antagonists (nutlin-3a), vaccines, targeted nanoparticles and immunomodulators.

All these compounds were reviewed in Campisi, J. From discoveries in ageing research to therapeutics for healthy ageing. Nature, (2019), 571(7764), 183-192*; Chaib et al (2022);* Dolgin, E. Send in the senolytics. Nat Biotechnol, (2020), 38(12), 1371-1377*; and Kirkland* & *Tchkonia (2020).*

The most widely investigated senolytic approaches involve navitoclax (N) and the combination of dasatinib plus quercetin (D+Q) *(*Cang, S. et al. ABT-199 (venetoclax) and BCL-2 inhibitors in clinical development. Journal of Hematology & Oncology, (2015), 8, 129*; Chang et al. (2016);* Hickson, L. et al. Senolytics decrease senescent cells in humans: Preliminary report from a clinical trial of Dasatinib plus Quercetin in individuals with diabetic kidney disease. EBioMedicine, (2019), 47, 446-456*; Tse et al., 2008).* In addition, the replacement of quercetin by fisetin has also been suggested *(Chaib et al., (2022);* Zhu, Y. et al. New agents that target senescent cells: The flavone, fisetin, and the BCL-XL inhibitors, A1331852 and A 1155463. Aging, (2017), 9(3), 955-963*).* However, the off-target effects of navitoclax, which causes thrombocytopenia limits its therapeutic application in its native form, while the long-term effects of D+Q or D+F warrant further investigations.

Despite successful preclinical proofs-of-concept for senolytics, their potential application in the clinic is hampered by their associated toxicities or inefficiency towards all senescent cells (i.e., there is no pan-senoloytic). There are no senolytic drugs marketed for clinical use yet.

The authors of the present invention have identified and improved a venom-derived protein from a marine source with senolytic and senomorphic properties.

Sticholysin I (aka Tvs1 throughout the application) is a marine venom-derived protein from the Caribbean Sea anemone *Stichodactyla helianthus* with an identified sequence of 176 aa. Tvs1 belongs to a family of proteins known to be potent pore-forming toxins produced by marine organisms (sea anemones), leading to cell death by osmotic shock (i.e., cytolytic activity). This family forms pores in membranes that contain sphingomyelin, cholesterol, or other lipids. Pore formation involves recognition of membrane sphingomyelin, binding to the membrane accompanied by the transfer of the N-terminal region to the lipid-water interface and oligomerization of three to four monomers. This pore is selective for monovalent cations *(*Hervis, Y. P., et al. Architecture of the pore forming toxin sticholysin I in membranes. Journal of Structural Biology, (2019), 208(1), 30-42*; Palacios-Ortega, J., et al (2020);* Tejuca, M., et al. Construction of an immunotoxin with the pore forming protein Stl and ior C5, a monoclonal antibody against a colon cancer cell line. International Immunopharmacology, (2004), 4(6), 731-744*).*

Binding of Tvs1 to lipid monolayer is dependent on the palmitoyl-oleyl-phosphatidylcholine (POPC) and sphingomyelin content. This has been proven only in artificial membranes (unilamellar vesicles) *(*Alvarez Valcarcel, C. et al. Effects of lipid composition on membrane permeabilization by Sticholysin I and II, two cytolysins of the sea anemone Stichodactyla helianthus. Biophysical Journal, (2001), 80(6), 2761-2774*;* Castrillo, I., et al. Specific interactions of sticholysin I with model membranes: An NMR study. Proteins: Structure, Function, and Bioinformatics, (2010), 78(8), 1959-1970*; Hervis et al. (2019);* Palacios-Ortega, J., et al. Sticholysin, Sphingomyelin, and Cholesterol: A Closer Look at a Tripartite Interaction. Biophysical Journal, (2019), 116(12), 2253-2265*;* Tejuca, M., et al. Mechanism of membrane permeabilization by sticholysin I, a cytolysin isolated from the venom of the sea anemone Stichodactyla helianthus. Biochemistry, (1996), 35(47), 14947-14957). Data in unilamellar vesicles suggest that membrane fluidity and composition provide a suitable environment for Tvs1-lipid binding and subsequent pore formation *(Palacios-Ortega et al., (2019);* Pedrera, L., et al. The Important Role of Membrane Fluidity on the Lytic Mechanism of the α-Pore-Forming Toxin Sticholysin I. Toxins, (2023), 15(1), 80*).* It has been suggested that function of proteins in model systems can significantly differ to their behaviour in natural lipid bilayers.

Tvs1 is haemolytic in human, sheep, and guinea pig erythrocytes. However, Tvs2 (a 93% homology-analogue of Tvs1) is more haemolytic than Tvs1 *(*Huerta, V., et al. Primary structure of two cytolysin isoforms from Stichodactyla helianthus differing in their hemolytic activity. Toxicon, (2001), 39(8), 1253-1256*;* Lanio, M. E., et al. Purification and characterization of two hemolysins from Stichodactyla helianthus. Toxicon, (2001), 39(2), 187-194*).*

Only a few studies have documented the interaction of Tvs1 with nucleated cells and their implications for intracellular processes. The cytotoxicity of the native form of Tvs1 has been studied in human T-acute lymphoblastic leukemia cells (CEM), chronic myelogenous leukemia (K562), human myelocytic leukemia cells (HL-60), peripheral mononuclear cells, human breast carcinoma lines (MDA-MB-231 and MDA-MB-134), and human colorectal cancer cells (SW948) *(*Avila, A. D. et al. A new immunotoxin built by linking a hemolytic toxin to a monoclonal antibody specific for immature 7 lymphocytes. International Journal of Cancer, (1988), 42(4), 568-571*;* Avila, A. D., et al. A carcinoembryonic antigen-directed immunotoxin built by linking a monoclonal antibody to a hemolytic toxin. International Journal of Cancer, (1989), 43(5), 926-929; *Tejuca et al., 2004).*

Now, the authors of the present invention, after a significative experimental effort, have shown that this venom derived protein (Tvs1), previously known for its anticancer activity, has senolytic and senomorphic properties.

Further, they have modified the sequence of Tvs1, obtaining a new peptide (Tvs1G) and increased by 23 times its senolytic index in comparison to the native protein (Tvs1). Compared to other senolytics, Tvs1G presents the highest senolytic index and potency, with a similar senolytic spectrum to navitoclax.

These new compounds have relevant clinical and cosmetic applications, providing pharmaceutical and cosmetic compositions for use in the prevention and treatment of aging and age-related diseases.

### BRIEF DISCUSSION OF THE FIGURES

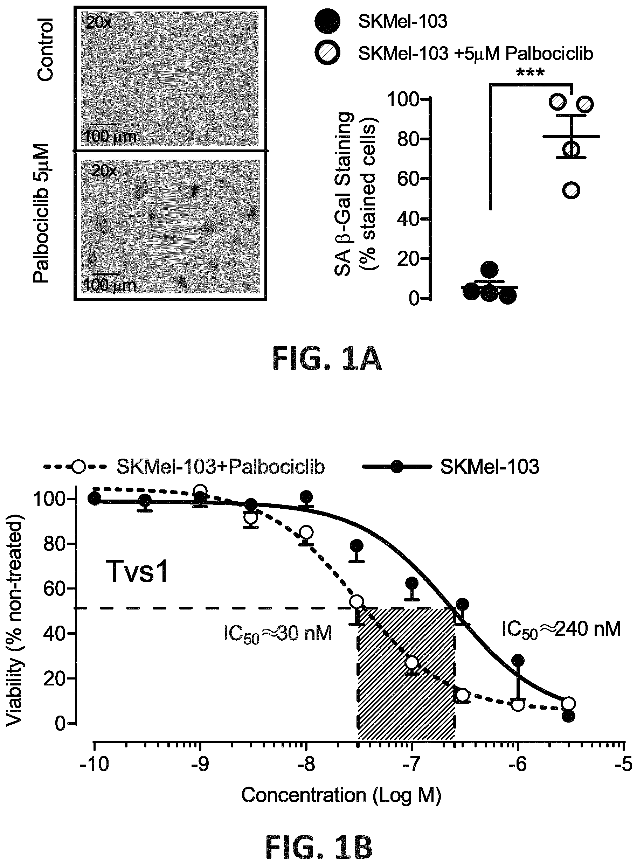

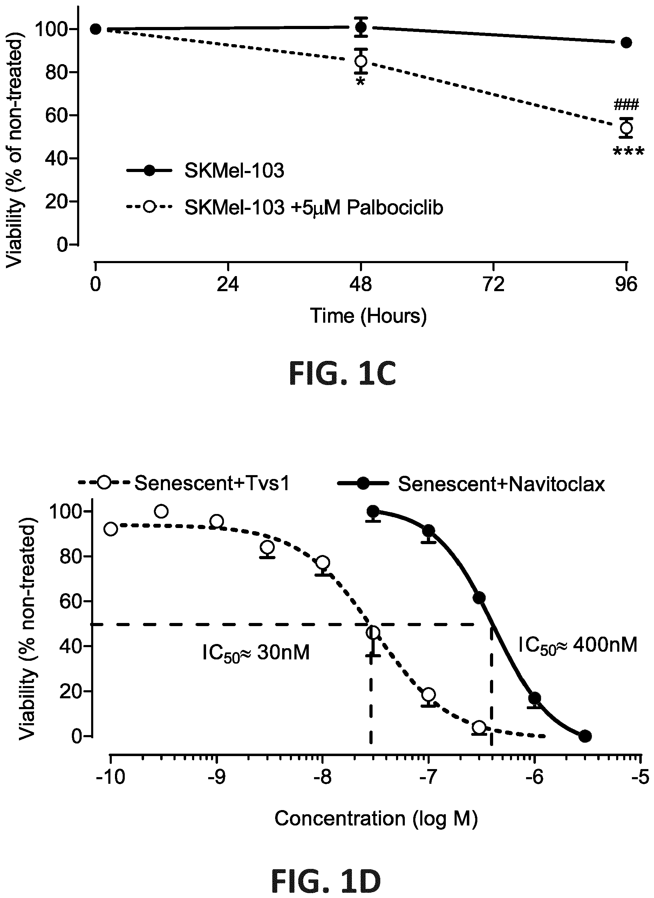

**Figure 1**. (A) Representative images (left) and quantification (right) for the senescent-associated β-galactosidase (SA β-Gal) staining in SKMel-103 cells under control conditions or treated with palbociclib (5µM, 7 days). (B) Concentration-dependent response curve for the effects of a 48h treatment with Tvs1 in the viability of proliferative SKMel-103 cells (black, control) and SKMel-103 treated with palbociclib (5µM, 7 days). (C) Chart illustrates the time-dependent changes in viability induced by low concentrations of Tvs1 (10nM) in control (black) and palbociclib-treated SKMel-103 (5µM, 7 days, stripped). (D) Comparison of the potency of Tvs1 (stripped) and navitoclax (black) analysed by viability assays after 48h of treatment with the indicated concentrations. Data are shown as mean ± SEM. n = 3-6. *p < 0.05 **p < 0.01 and ***p < 0.001 vs control cells, and ### p<0,001 vs 48h

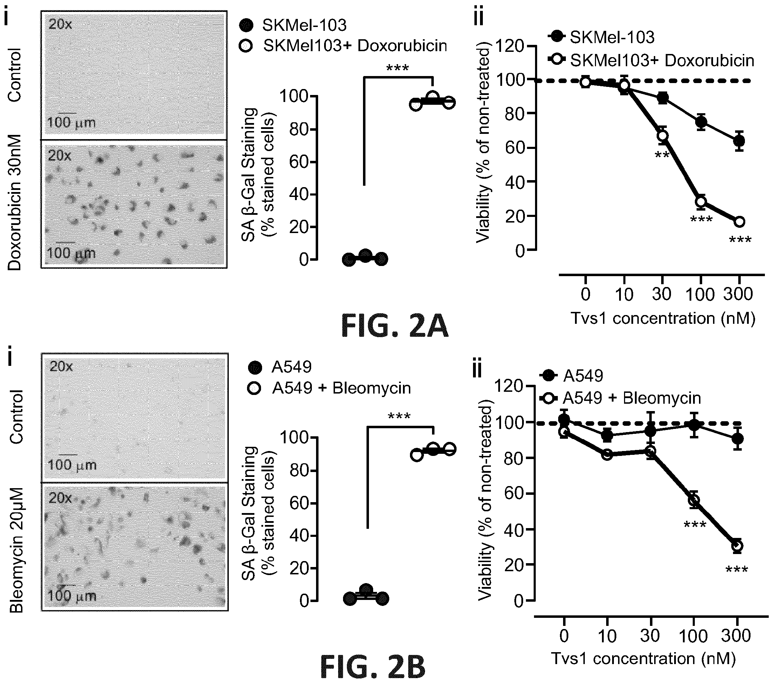

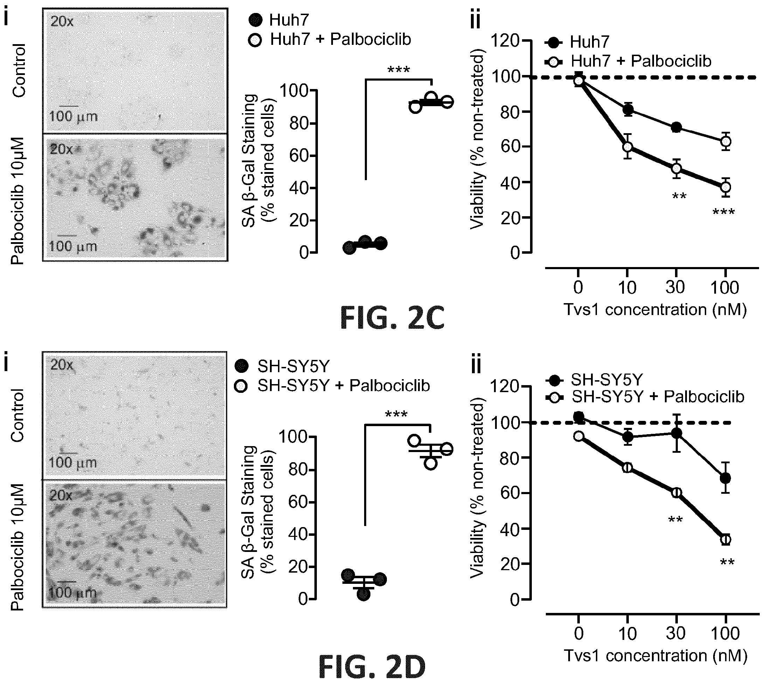

**Figure 2****.** Representative images (i) for the senescent-associated β-galactosidase (SA β-Gal) staining (left), quantification of the stained population (right) and (ii) the concentration-dependent response curve for the effects of Tvs1 in the viability of proliferative (black) and senescent (white) (**A**) SKMel-103 cells treated with doxorubicin (30nM, 7 days), (**B**) A549 cells treated with bleomycin (20µM, 5 days), (C) Huh7 cells treated with palbociclib (10µM, 7 days) and (**D**) SH-SY5Y cells treated with palbociclib (10µM, 7 days). Data are shown as mean ± SEM. n = 3-5. **p < 0.01 and ***p < 0.001 vs control cells.

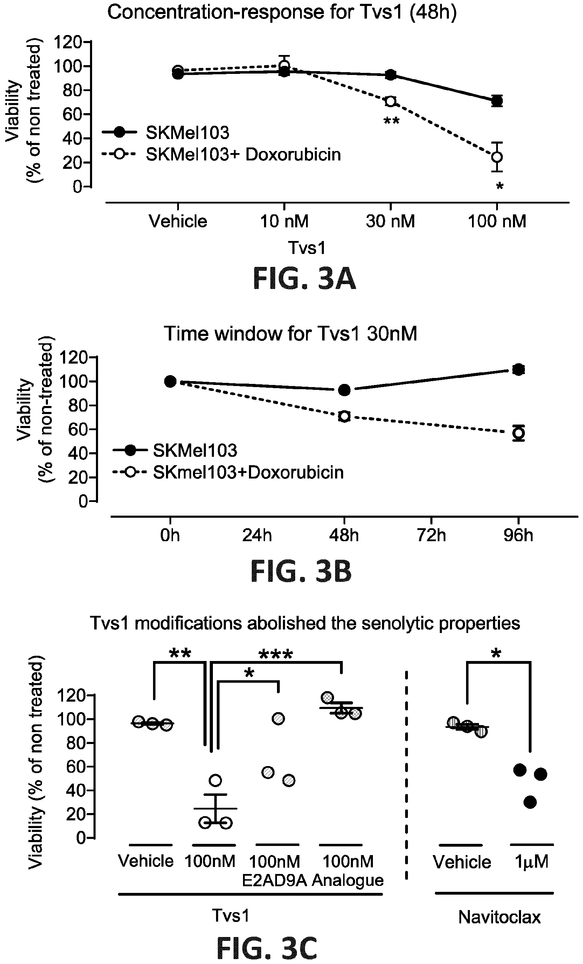

**Figure 3**. (A) Viability assays for the effect of increasing concentrations of Tvs1 in proliferative SKMel103 cells and senescent SKMel-103 cells treated with doxorubicin (30nM, 5 days). (**B**) Chart illustrates the time-dependent changes in viability induced by low concentrations of Tvs1 (30nM) in control (black) and doxorubicin-treated SKMel-103 (30nM, 5 days, dashed line). (**C**) Dot-plot indicates viability of senescent SKMel-103 (doxorubicin 30M, 5 days) treated for 48h with the indicated treatments. Data are shown as mean ± SEM. n = 2-3. *p < 0.05, **p < 0.01 and ***p < 0.001 vs control cells.

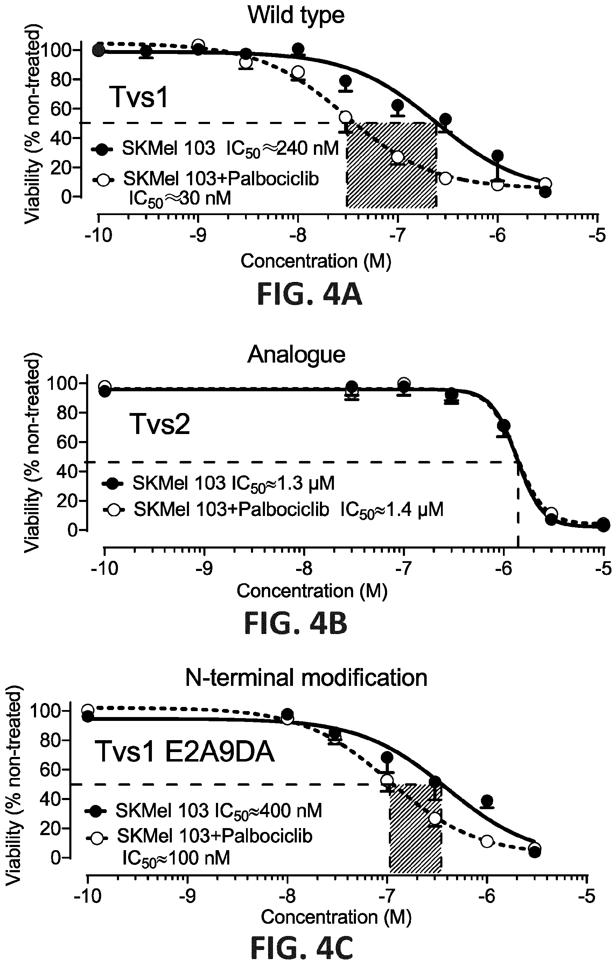

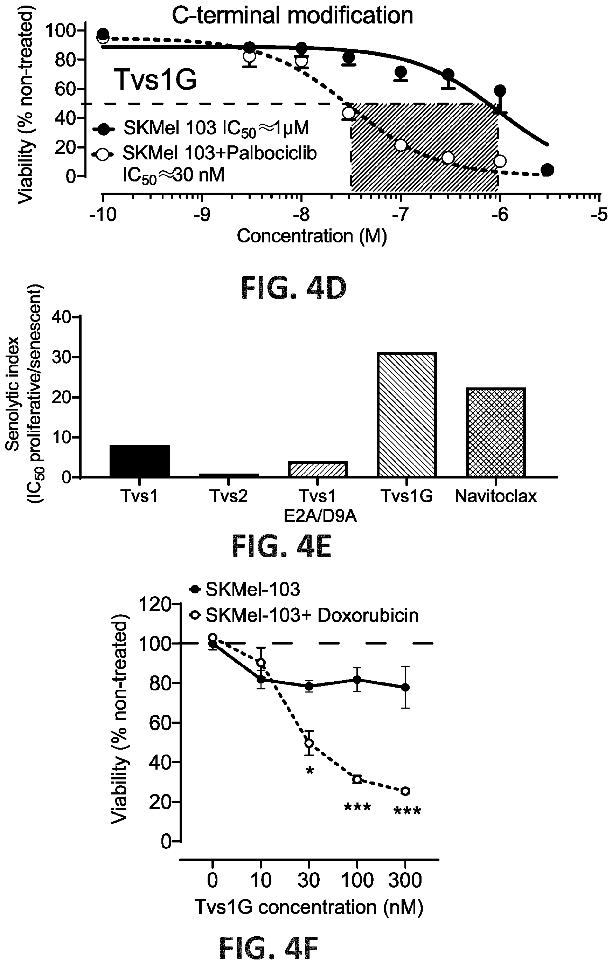

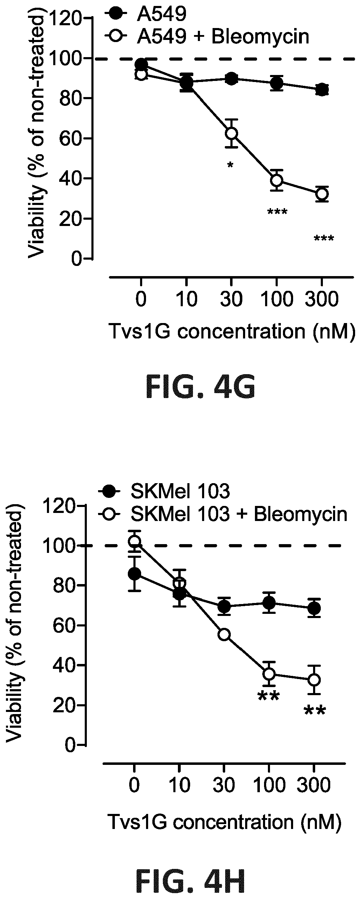

**Figure 4****.** Concentration-dependent response curve shows the viability of a 48h treatment with Tvs1 wild type **(A),** Tvs2 (**B**), Tvs1 E2AD9A (C) and Tvs1G (D) in proliferative SKMel-103 cells (black, control) and SKMel-103 treated with palbociclib (5µM, 7 days). (**E**) Chart illustrates the senolytic index calculated as a ratio of the IC50 in proliferative and senescent cells. (**F**) Concentration-dependent response curve on the viability of a 48h treatment with Tvs1G in proliferative (black, control) and SKMel-103 + 30nM doxorubicin, (**G**) A549+ 20µM bleomycin and (H) SKMeI-103+ 5µM bleomycin. Data are shown as mean ± SEM. n = 3-5 *p < 0.05, **p < 0.01 and ***p < 0.001 vs control cells

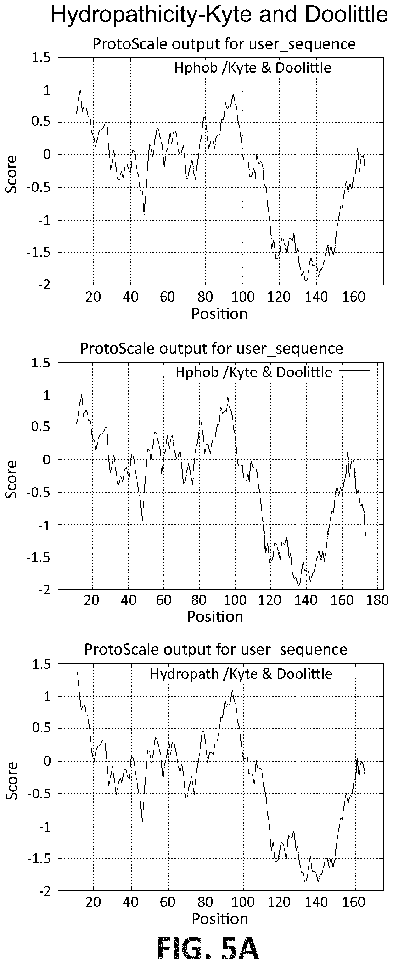

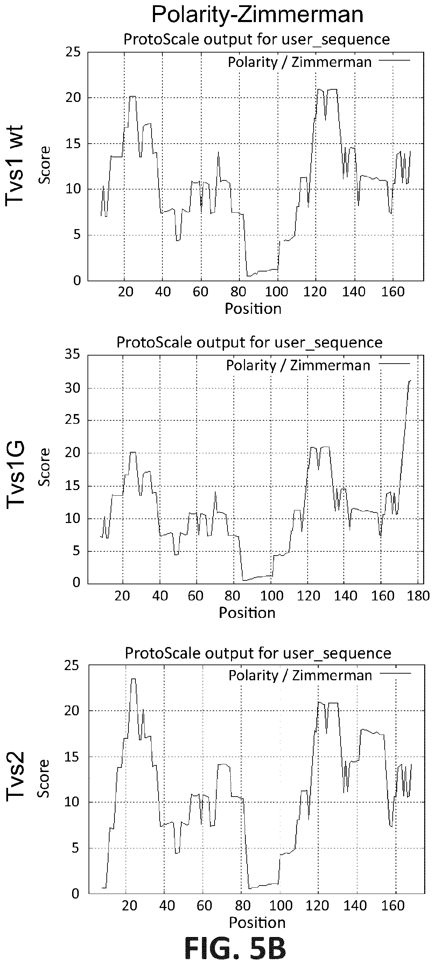

**Figure 5****.** Hydropathicity of Kyte and Doolittle (**A**) and polarity of Zimmerman (**B**) calculated for Tvs1 wild type (Tvs1 wt), Tvs1G and the analogue Tvs2.

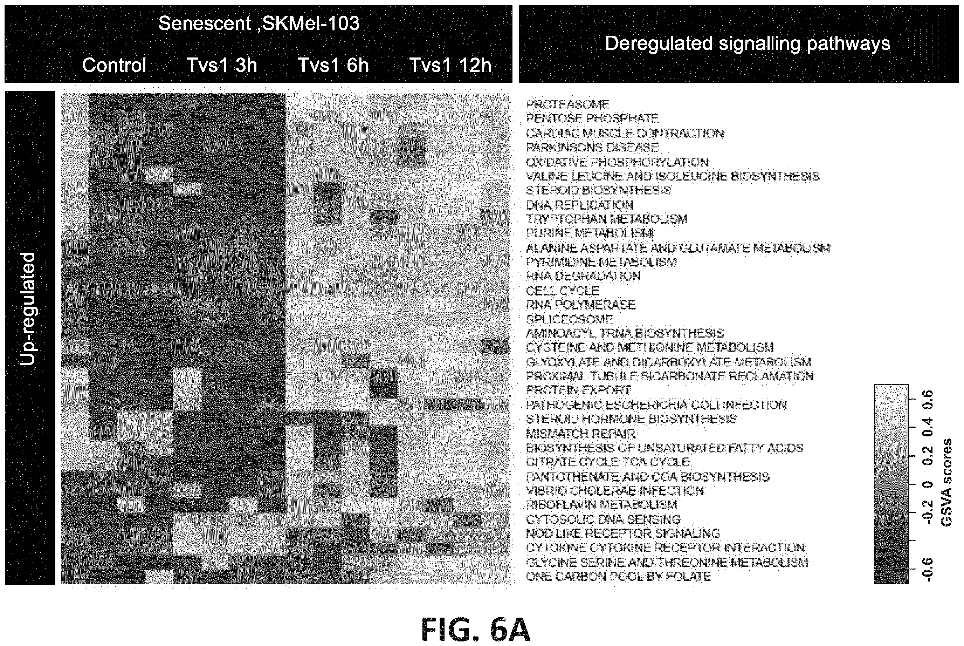

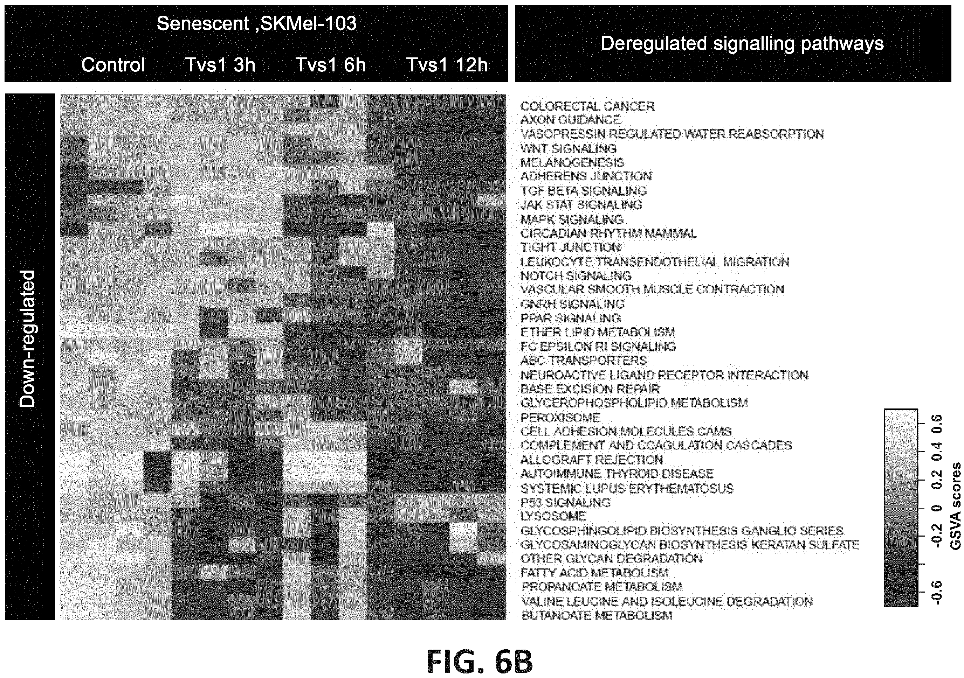

**Figure 6****.** Gene-set enrichment analysis (GSVA) indicates the most significantly deregulated signalling pathways in senescent SKMel-103 cells (palbociclib 5 µM, 7 days) under control conditions (left) or after 3-6-12h treatment with Tvs1G 100nM. Four replicates were done per treatment.

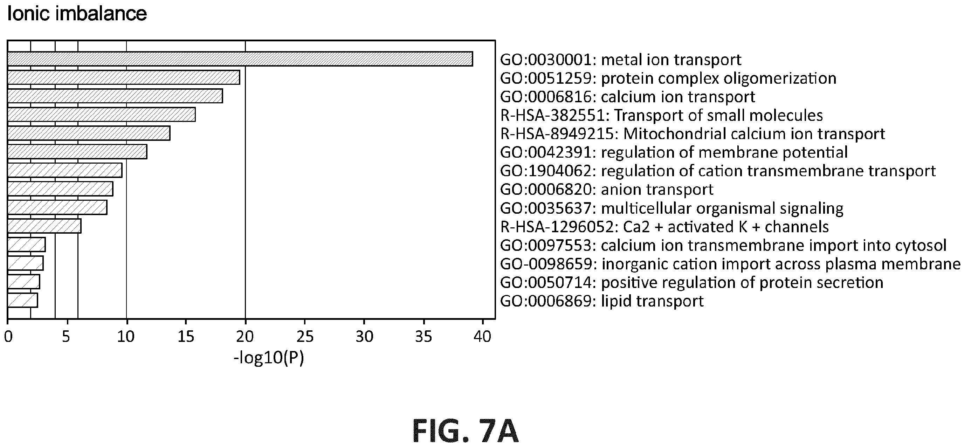

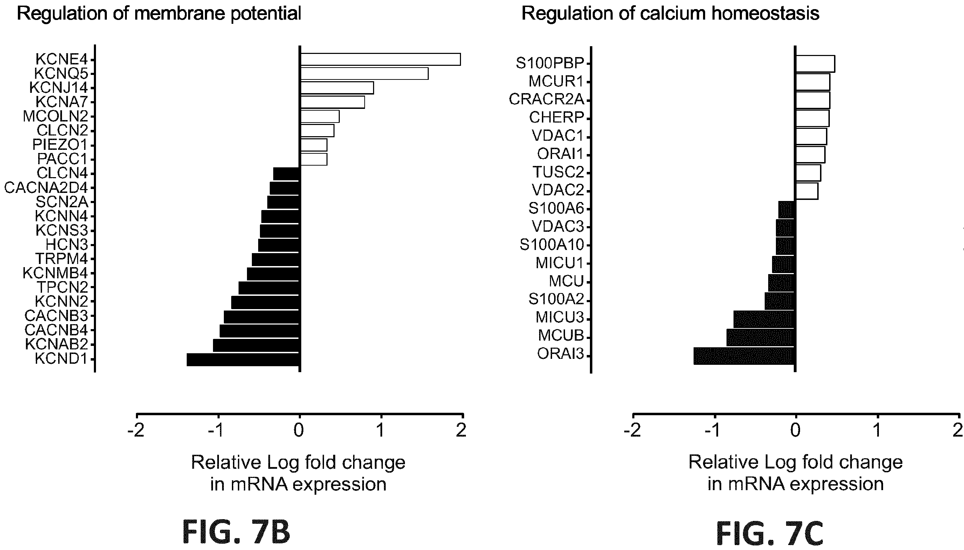

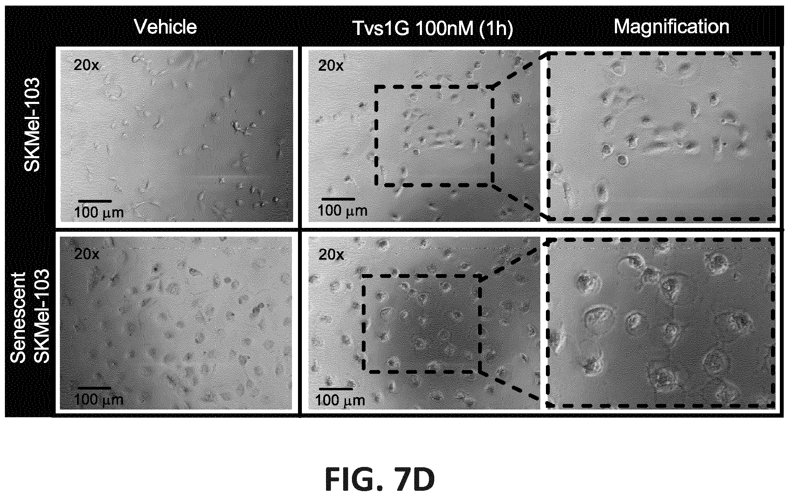

**Figure 7****.** (**A**) The most significant Gene ontology (GO) biological processes associated with the identified differentially expressed genes. The vertical axis represents the GO category, and the horizontal axis represents the -Log10(P-value) of the significant GO terms. Bar chart indicates the relative fold change for genes involved in the regulation of (**B**) the membrane potential and (C) calcium homeostasis following a 12h treatment with 100nM Tvs1G in senescent SKMel-103. The vertical axis indicates the gene, and the horizontal axis represents the -Log10(P-value). Greater -Log10(P-value) scores correlated with increased statistical significance. (**D**) Representative images of proliferative (up) or senescent (down) SKMel103 treated with vehicle or Tvs1G 100nM after 1 hour of incubation.

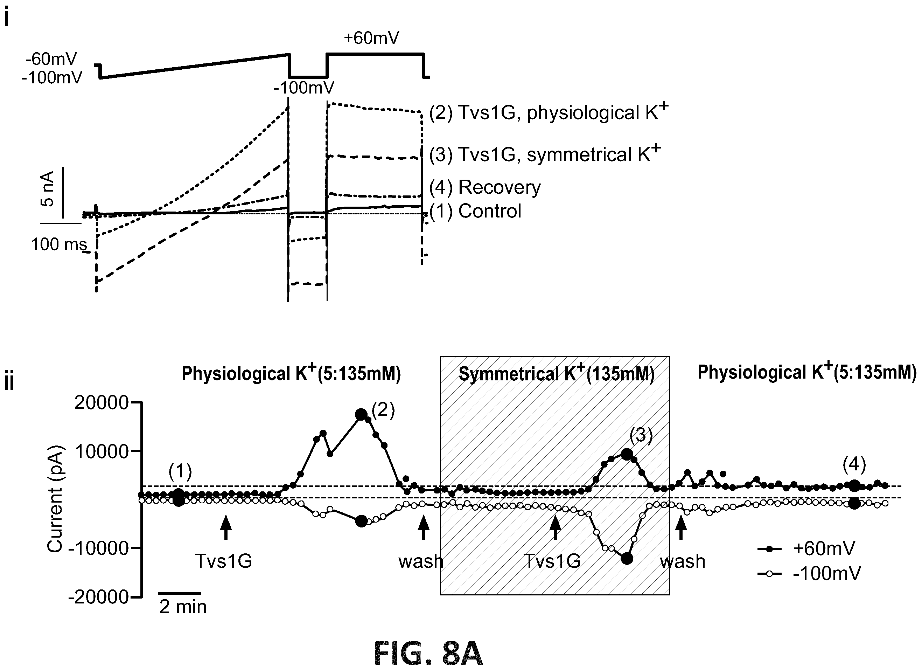

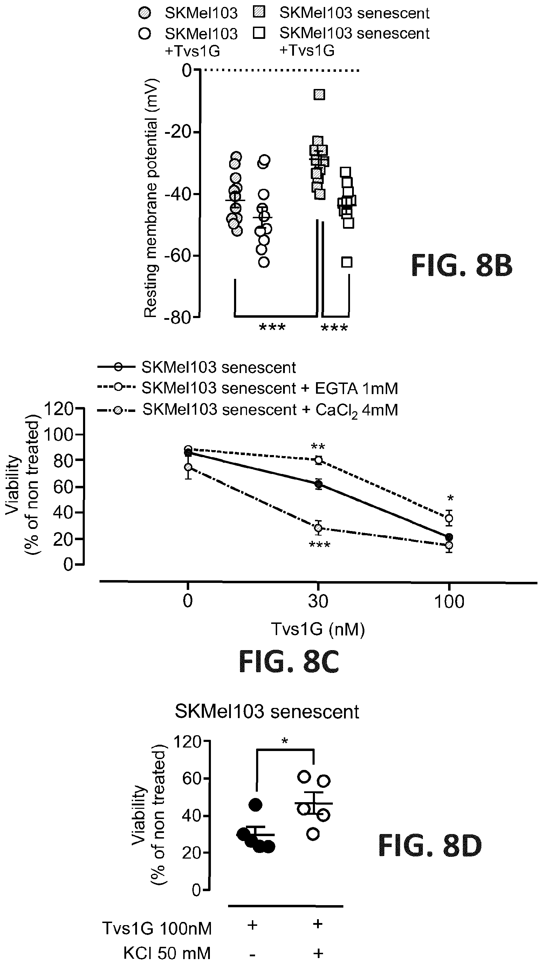

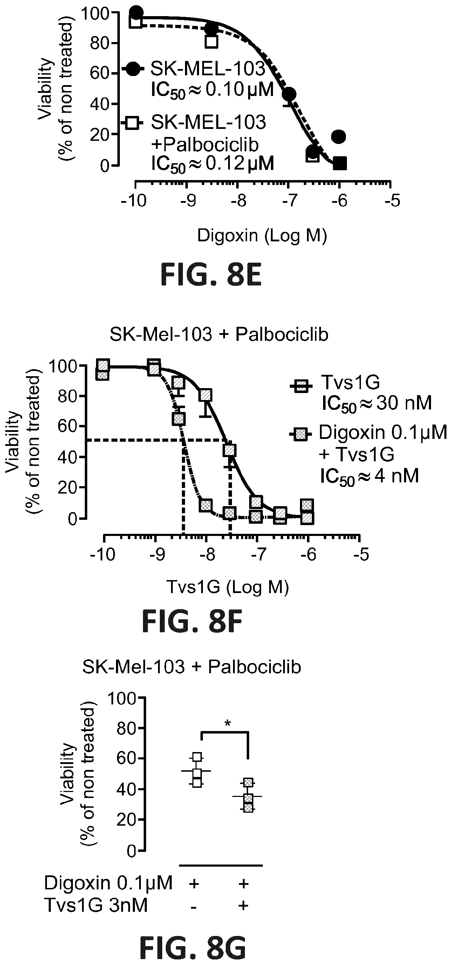

**Figure 8****.** (**A**) Representative records (i) for voltage-clamp ramp and step protocol and (ii) the plotted time course for the currents recorded at the steady state of the voltage step to -100 mV (white) and +60 mV (black). Numbers indicate matching traces in i and time points in ii. (1) before the addition of 10nM Tvs1G, (2) at the maximal effects under asymmetrical K⁺, (3) at the maximal effects under symmetrical K+ and (4) 8 minutes after washing off Tvs1G in senescent induced via palbociclib SKMEL-103 cells. (B) Dot plot indicates resting membrane potential in proliferative SKMel-103 cells (black) and palbociclib-induced senescent SKMel-103 cells (white) treated with vehicle or Tvs1G 100nM for 15-20h. (C) Viability at 48h of treatment with Tvs1G in senescent SKMel-103 cells under control conditions (black) and supplemented with 1mM EGTA, 4mM CaCl2 or **(D)** 50mM KCI. (**E**) Concentration-dependent response curve showing the viability of a 48h treatment with digoxin in SKMel-103 (black, control) and senescent SKMel-103 cells. (**F**) Normalized viability of senescent SKMel103 cells following 48h treatment with increasing concentrations of Tvs1G alone or in combination with 0.1µM digoxin. (**G**) Dot-plot indicates viability of senescent SKMel-103 treated for 48h with the indicated treatments. Results are expressed as mean ± SEM, n = 3-9. *p<0.05;**p<0.01 and ***p < 0.001 for the indicated comparisons.

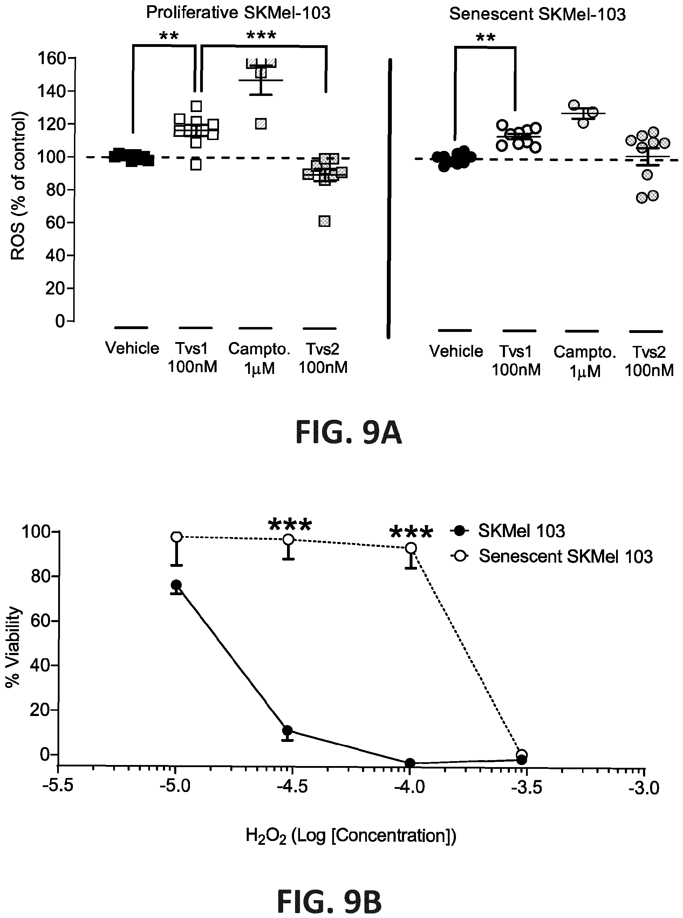

**Figure 9**. (**A**) Dot plot for the detection of intracellular reactive oxygen species (ROS) after 24h of the indicated treatments, determined with dihydrofluorescein diacetate by flow cytometry and expressed as percentage of the mean fluorescence of control. (B) Concentration-dependent response curve for the effects of tert-butyl hydroperoxide in the viability of proliferative (black) and senescent (white) SKMel-103 cells treated with palbociclib (5µM, 7 days). Data are shown as mean ± SEM. n = 3-9. **p < 0.01 and ***p < 0.001 vs control cells.

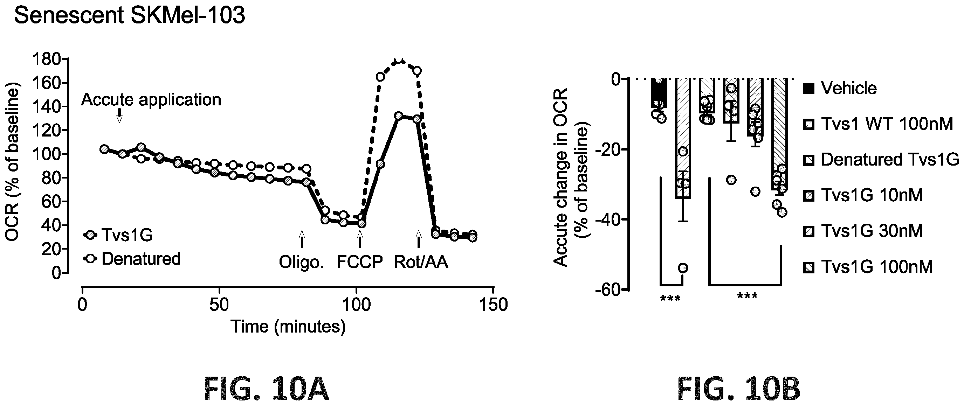

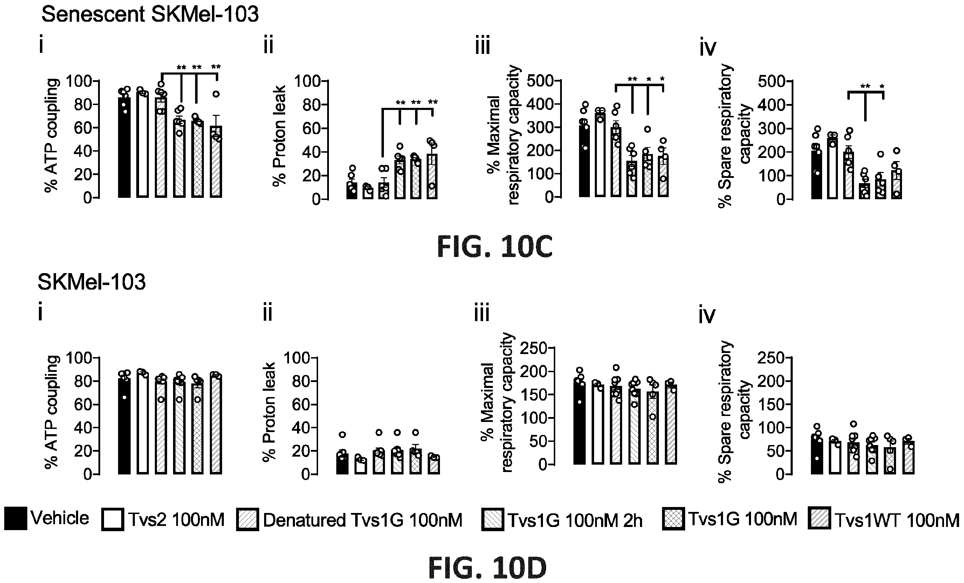

**Figure 10****.** (**A**) Representative traces in senescent SKMel-103 cells for normalized oxygen consumption rates (OCR) to show real time measurements. Arrows signal addition of compounds to interrogate the respiratory parameters summarized in the bar charts. Plot indicates (**B**) the registered acute change in OCR normalized to baseline and the specific mitochondrial consumption parameters normalized to mitochondrial respiration and calculated for (C) palbociclib-induced senescent SKMel-103 cells and (D) proliferative SKMel-103 cells following the indicated treatments. Data are shown as mean ± SEM. n = 3-6. *p < 0.05, **p < 0.01 and ***p < 0.001 for the indicated comparisons.

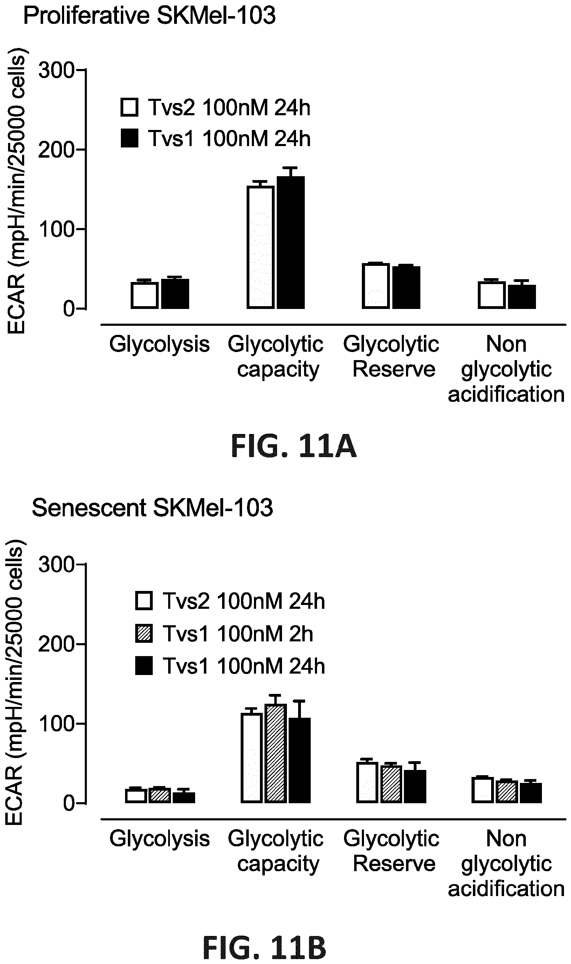

**Figure 11****.** Bar chart indicates Seahorse analysis for extracellular-acidification rates (ECAR) in (**A**) proliferative SKMel-103 cells following the indicated treatments and (**B**) palbociclib-induced senescent SKMel-103 cells. Data are shown as mean ± SEM. n = 3

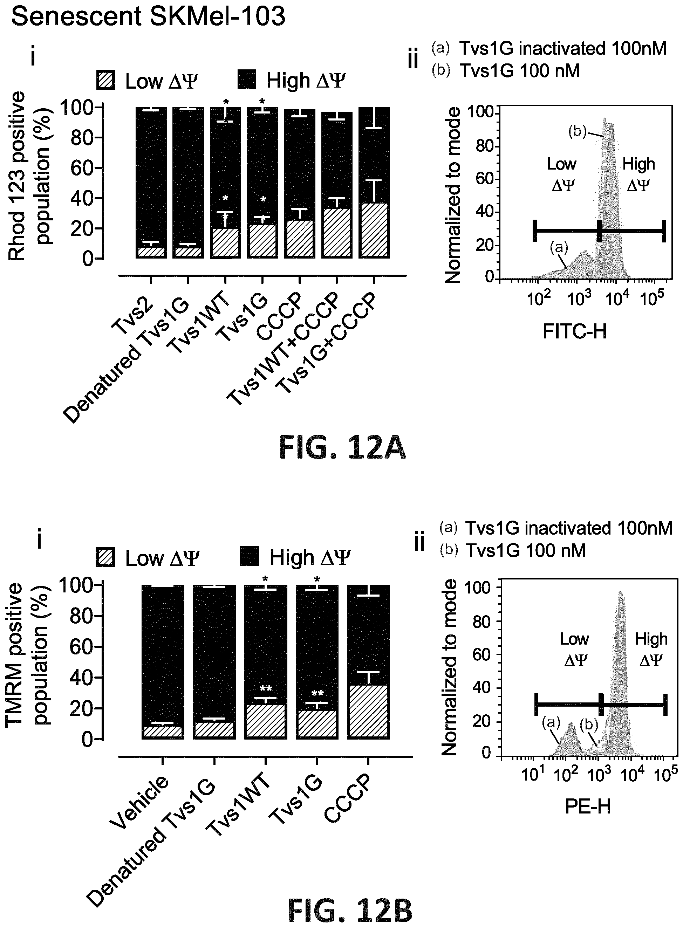

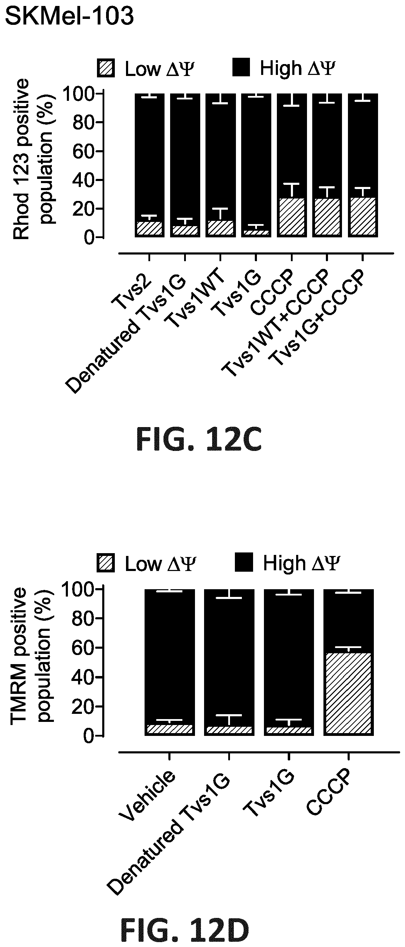

**Figure 12****.** Flow cytometry measurements of mitochondrial membrane potential determined with Rhodamine123 and TMRM dyes for (**A,B**) palbociclib-induced senescent SKMel-103 cells and (C,D) proliferative SKMel-103 cells following a 24h incubation with 100nM Tvs1, 100nM Tvs1G, 100nM Tvs2, 100nM denatured Tvs1G or 2h with 50 µM CCCP (carbonyl cyanide m-chlorophenyl hydrazone, protonophore). Data are shown as mean ± SEM. n = 6-9. *p < 0.05 and **p < 0.01 vs control

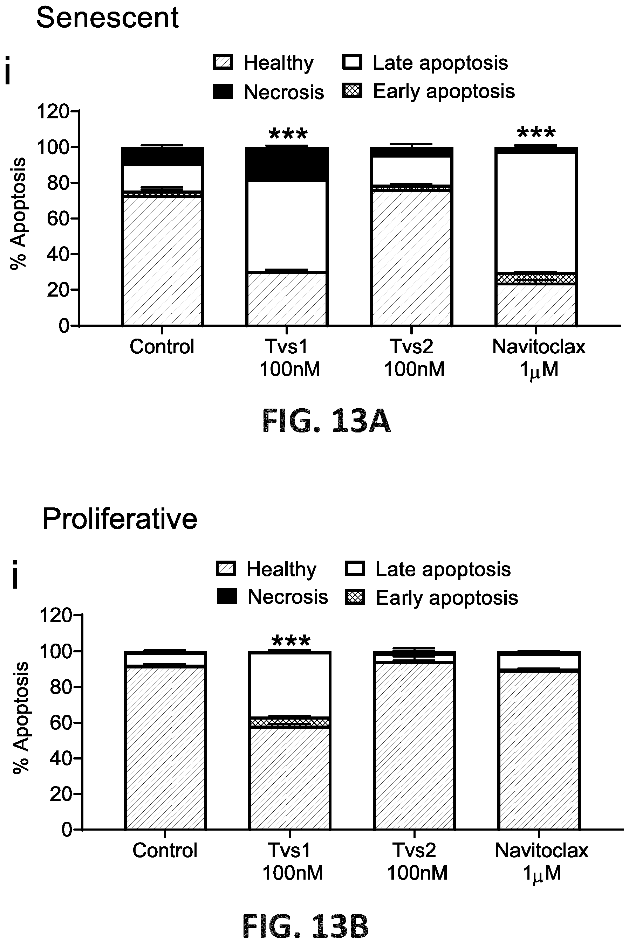

**Figure. 13****.** Annexin V-FITC detection of apoptosis in (**A**) palbociclib-induced senescent SKMel-103 cells and (**B**) proliferative SKMel-103 cells following a 48-h incubation with 100nM Tvs1 as compared to control conditions (n = 4). Data are shown as mean ± SEM. n = 3-9. ***p < 0.001 vs control cells.

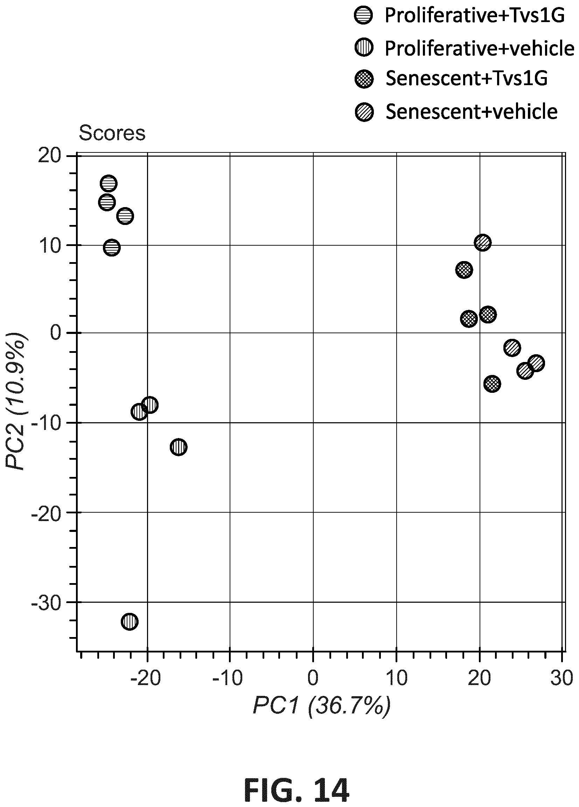

**Figure 14****.** Principal component analysis (PCA) scores for the individual samples of proliferative and senescent SKMel-103 cells treated with vehicle or Tvs1G 100nM for 12 h based on the lipid dataset standardized to the total lipid amount. N=4

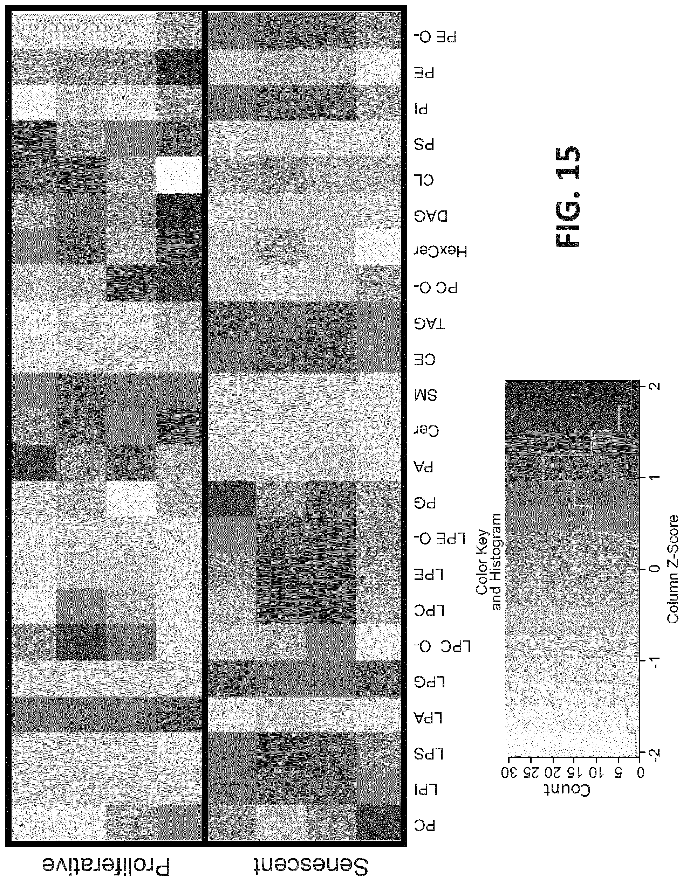

**Figure 15****.** Standardized score of representative members of major lipid classes in proliferative SKMel-103 cells and in palbociclib-induced senescent SKMel-103 (5µM, 7 days). Abbreviations: CE, Cholesterol esters; Cer, Ceramide; CL, Cardiolipin; DAG, Diacylglycerol; HexCer, Hexosylceramide; LPA, lyso-Phosphatidate; LPC O-, lyso-Phosphatidylcholine (-ether); LPE O-, lyso-Phosphatidylethanolamine (-ether); LPI, lyso-Phosphatidylinositol; LPS, lyso-Phosphatidylserine; PA, Phosphatidate; PC O-, Phosphatidylcholine (-ether); PE O-, Phosphatidylethanolamine (-ether); PG, Phosphatidylglycerol; PI, Phosphatidylinositol; PS, Phosphatidylserine; SM, Sphingomyelin; TAG Triacylglycerol. Relative abundances were standardized to the total lipid amount (mol%) per sample.

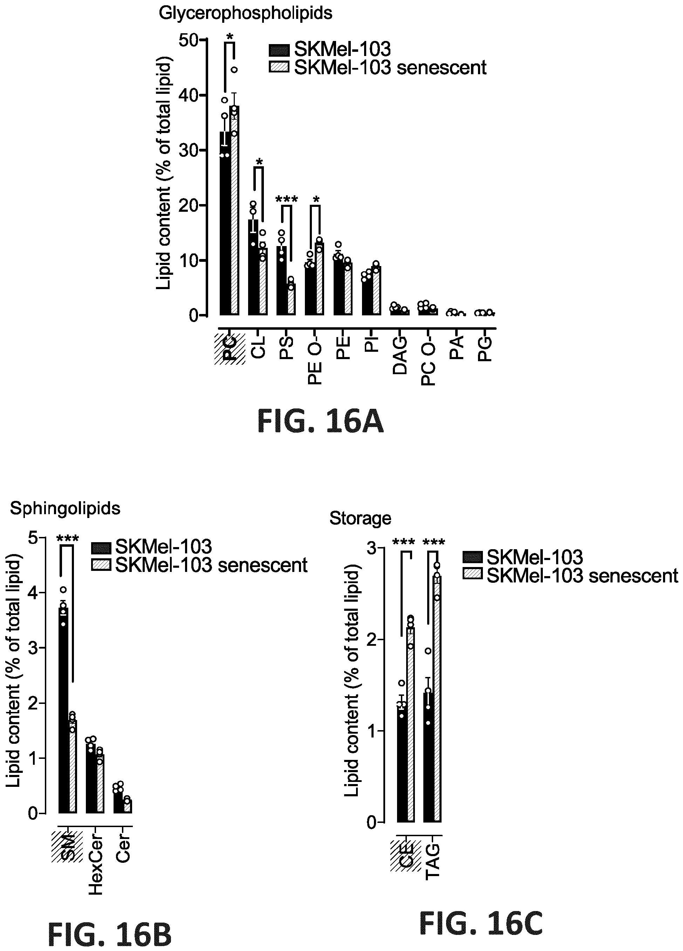

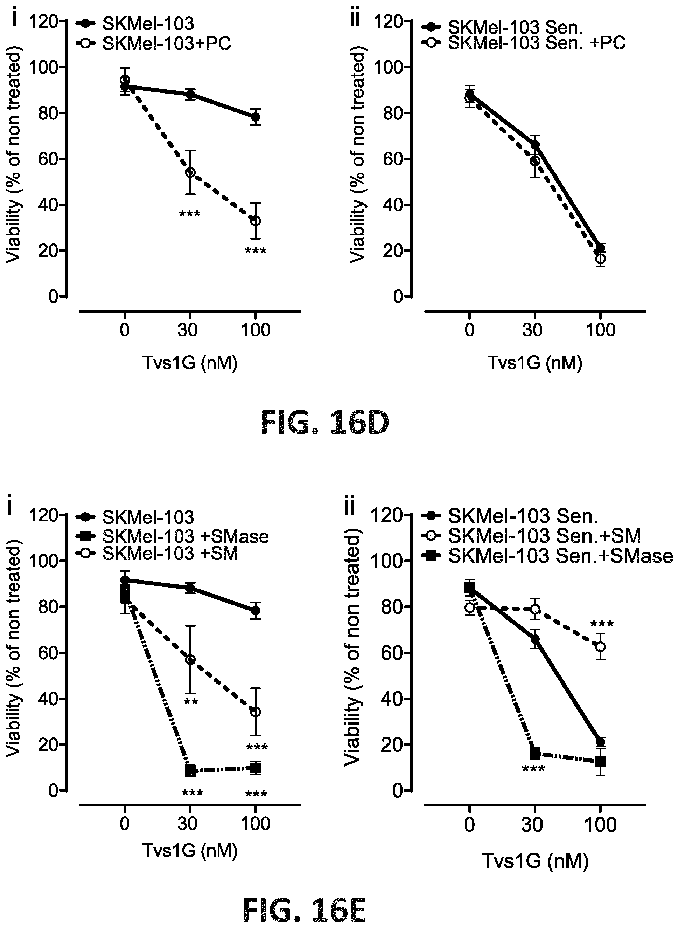

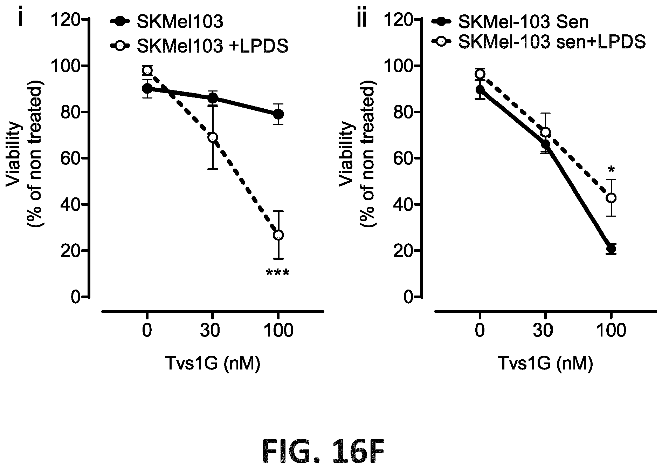

**Figure 16****.** Plots indicate lipid content standardized to total lipids (%mol) for (**A**) glyceraophospholipids, (**B**) sphingolipids and (**C**) storage lipids in proliferative SKMel-103 cells (black) and in palbociclib-induced senescent SKMel-103 (stripped, 5µM, 7 days). Concentration-dependent response at 48h of treatment with Tvs1G in the viability of (i) proliferative or (ii) senescent SKMel-103 cells under control conditions (black) and supplemented with (D) 100µM palmitoyl-2-oleoylphosphatidylcholine (PC), (E) 100µM sphingomyelin (SM) or 100mU sphyngomyelinase (SMAse) and (F) lipoprotein-depleted serum (LPDS). Abbreviations: CE, Cholesterol esters; Cer, Ceramide; CL, Cardiolipin; DAG, Diacylglycerol; HexCer, Hexosylceramide; PS, Phosphatidylserine; PG, Phosphatidylglycerol; PI, Phosphatidylinositol; PS, Phosphatidylserine; SM, Sphingomyelin; TAG Triacylglycerol. Relative abundances were standardized to the total lipid amount (mol%) per sample. Data are shown as mean ± SEM. n = 3-6. *p < 0.05, **p < 0.01 and ***p < 0.001 for the indicated comparisons.

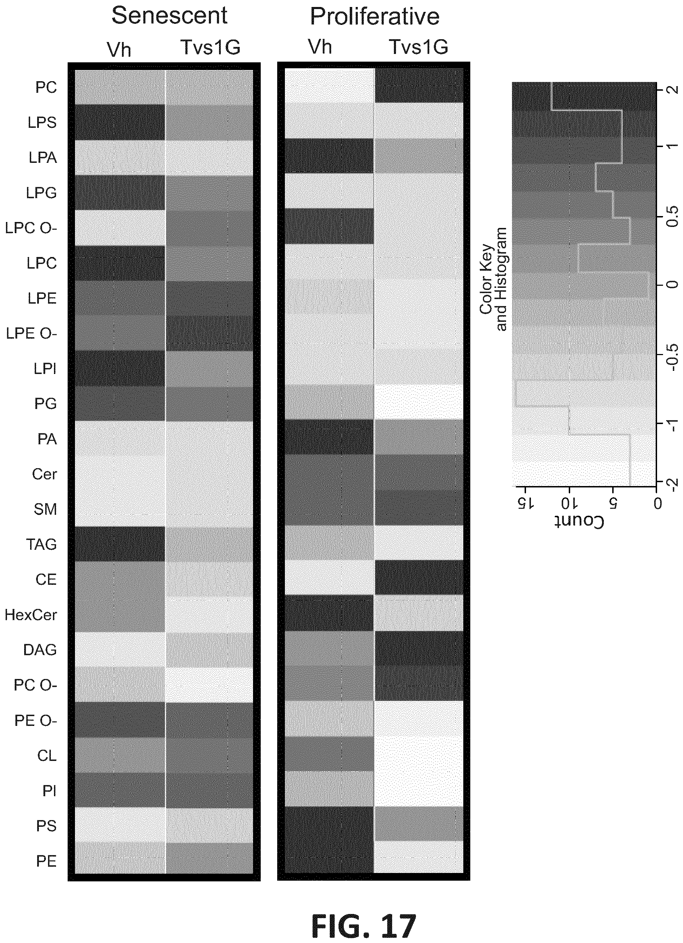

**Figure 17****.** Standardized score of representative members of major lipid classes during in proliferative SKMel-103 cells and in palbociclib-induced senescent SKMel-103 (5µM, 7 days) treated with vehicle (PBS 0,1%) or 100nM Tvs1G for 12h. Abbreviations: CE, Cholesterol esters; Cer, Ceramide; CL, Cardiolipin; DAG, Diacylglycerol; HexCer, Hexosylceramide; LPA, lyso-Phosphatidate; LPC O-, lyso-Phosphatidylcholine (-ether); LPE O-, lyso-Phosphatidylethanolamine (-ether); LPI, lyso-Phosphatidylinositol; LPS, lyso-Phosphatidylserine; PA, Phosphatidate; PC O-, Phosphatidylcholine (-ether); PE O-, Phosphatidylethanolamine (-ether); PG, Phosphatidylglycerol; PI, Phosphatidylinositol; PS, Phosphatidylserine; SM, Sphingomyelin; TAG Triacylglycerol. Relative abundances were standardized to the total lipid amount (mol%) per sample.

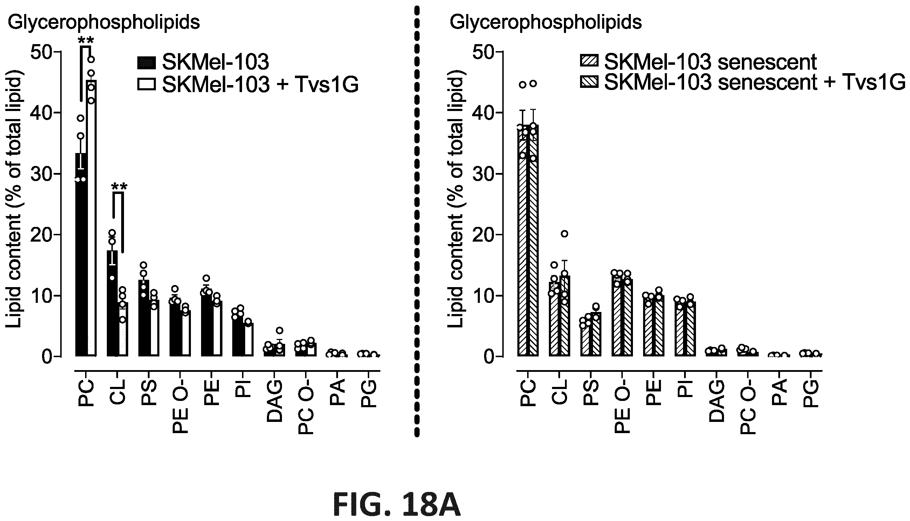

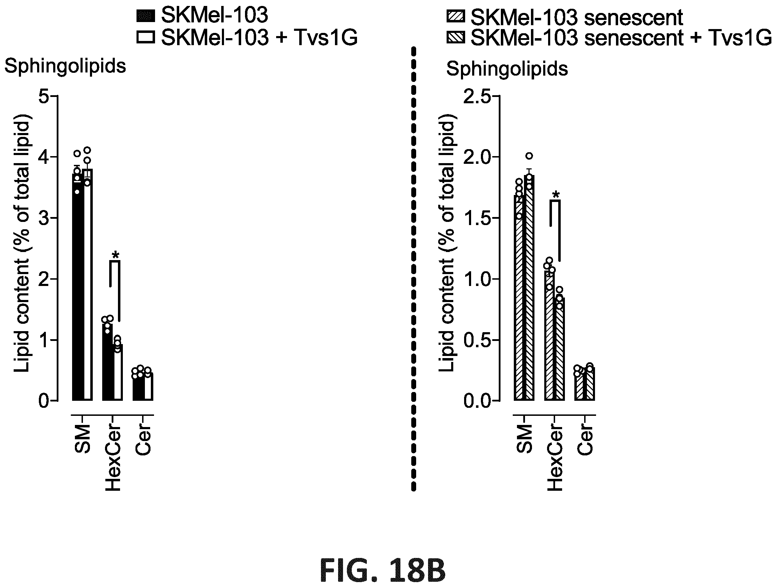

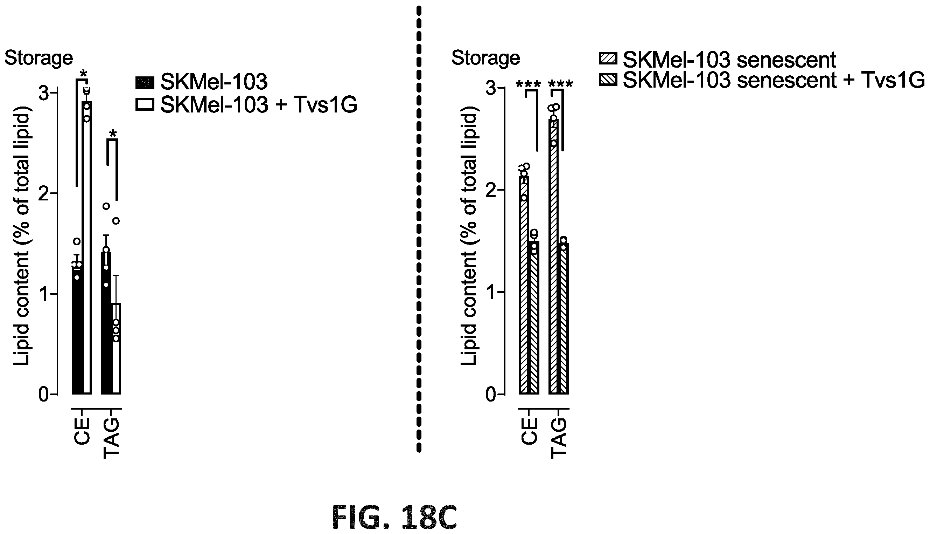

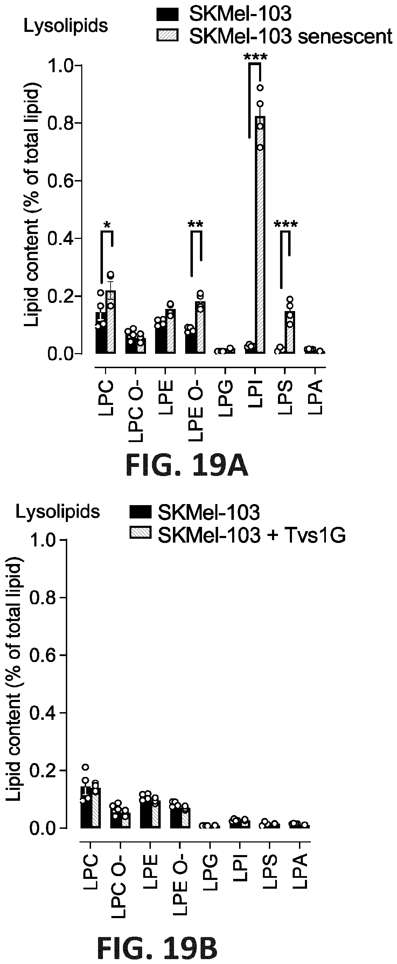

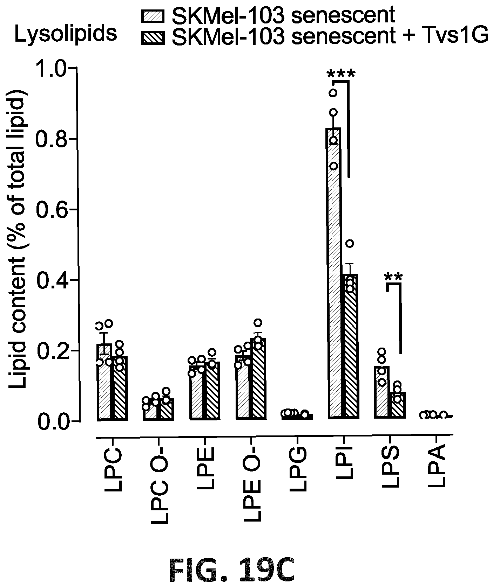

**Figure 18****.** Plots indicate lipid content standardized to total lipids (%mol) for (A) glyceraophospholipids, (**B**) sphingolipids and (**C**) storage lipids in proliferative SKMel-103 cells (left) and in palbociclib-induced senescent SKMel-103 (right, 5µM, 7 days) under control conditions (PBS 0,1%) or treated with 100nM Tvs1G for 12h. Abbreviations: CE, Cholesterol esters; Cer, Ceramide; CL, Cardiolipin; DAG, Diacylglycerol; HexCer, Hexosylceramide; PA, Phosphatidate; PC, Phosphatidylcholine; PE, Phosphatidylethanolamine ; PG, Phosphatidylglycerol; PI, Phosphatidylinositol; PS, Phosphatidylserine; SM, Sphingomyelin; TAG Triacylglycerol. Relative abundances were standardized to the total lipid amount (mol%) per sample. Data are shown as mean± SEM. n = 4. *p < 0.05, **p < 0.01 and ***p < 0.001 for the indicated comparisons. **Figure 19****.** Plots indicate lysophospholypids content standarized to total lipids (%mol) for the comparison (**A**) proliferative SKMel-103 cells vs palbociclib-induced senescent SKMel-103 (5µM, 7 days), (**B**) proliferative SKMel-103 cells or (C) palbociclib-induced senescent SKMel-103 following a 12 h treatment with vehicle (PBS 0,1%) or 100nM Tvs1G. Abbreviations: LPA, lyso-Phosphatidate; LPC O-, lyso-Phosphatidylcholine (-ether); LPE O-, lyso-Phosphatidylethanolamine (-ether); LPI, lyso-Phosphatidylinositol; LPS, lyso-Phosphatidylserine; PA, Phosphatidate; PC O-, Phosphatidylcholine (-ether); PE O-, Phosphatidylethanolamine (-ether). Relative abundances were standardized to the total lipid amount (mol%) per sample. Data are shown as mean ± SEM. n = 4. *p < 0.05, **p < 0.01 and ***p < 0.001 for the indicated comparisons.

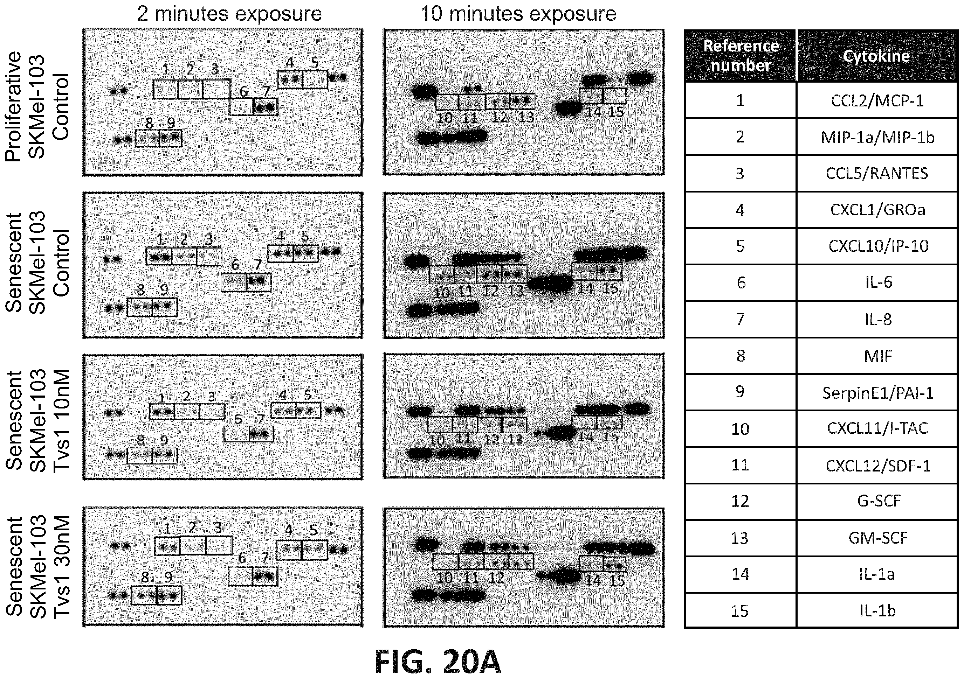

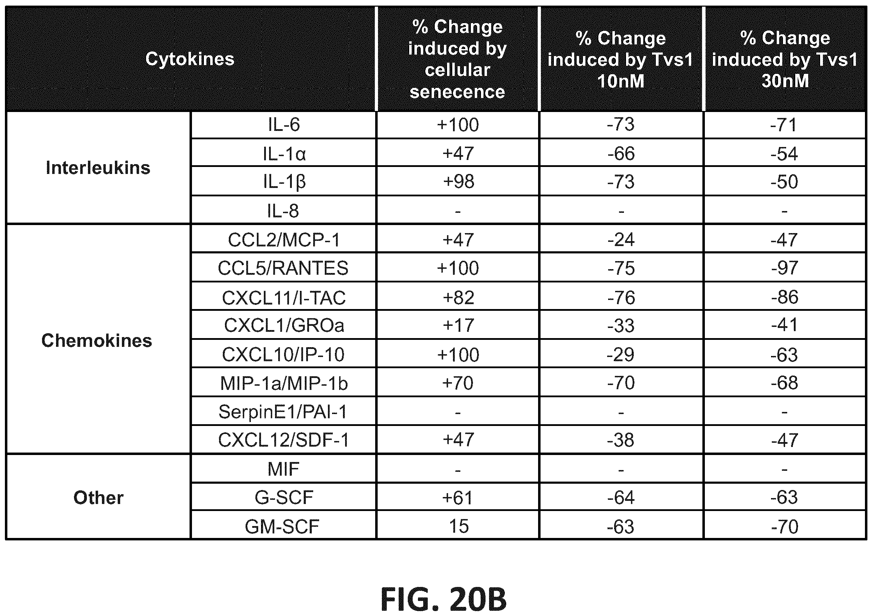

**Figure 20****.** (**A**) Images of arrays for the SASP secreted by proliferative SKMel-103 cells (control) as compared to senescent SKMel-103 cells (control, palbociclib 5µM, 7 days), and in senescent SKMel-103 cells treated with 10-30nM Tvs1 for 4 days. (B) Relative quantification of the arrays shown in A as a change in the percentage of cytokine detection. % Change induced by cell senescence=(Senescent raw intensity density - proliferative raw intensity density)/ Senescent raw intensity x100. % Change induced by Tvs1=(Tvs1-treated raw intensity density - senescent raw intensity density)/ senescent raw intensity x100.

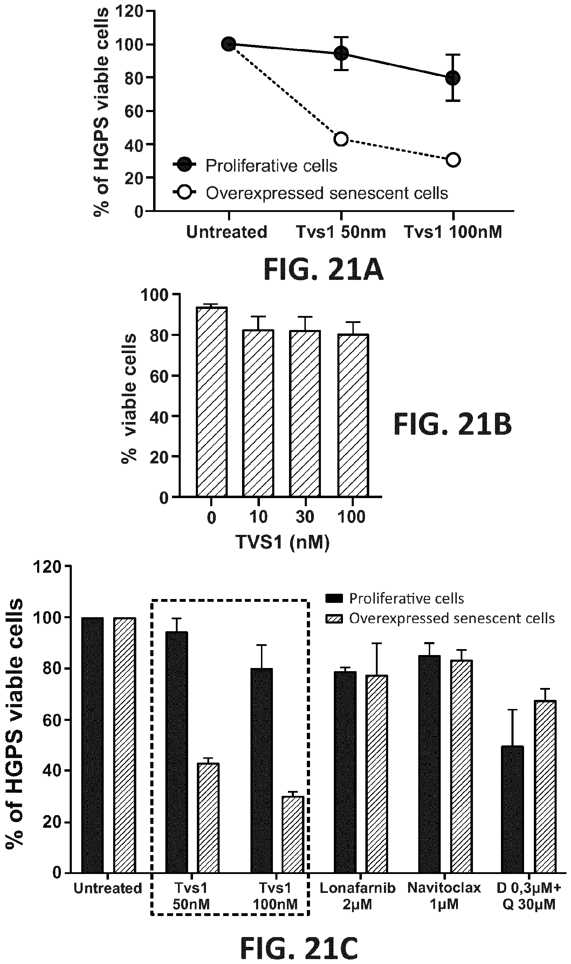

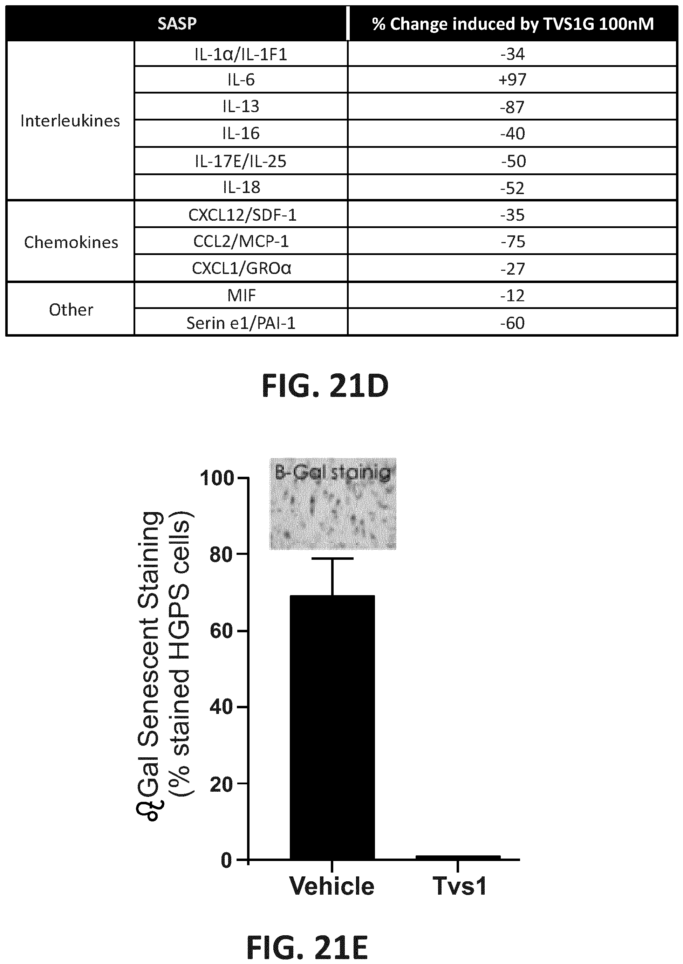

**Figure 21****.** (A) TVS1G preferentially targets overexpressed via Palbociclib senescent HGPS cells. (**B**) TVS1G is innocuous in human dermal fibroblasts. (C) Comparison of TVS1G with other known senolytics (navitoclax, D+Q; dasatinib+quercetin) and lonafarnib. Treatments were done after 48h of incubation using HGADFN127 and HGADFN178 cells. **(D)** TVS1G modulates SASP in progeria cells HGADFN127 cells. (E) Reduction of b-gal staining suggests that TVS1G diminishes senescence in progeria HGPS (AG06917K) cells. Data are shown as mean ± SEM. n = 3-6.

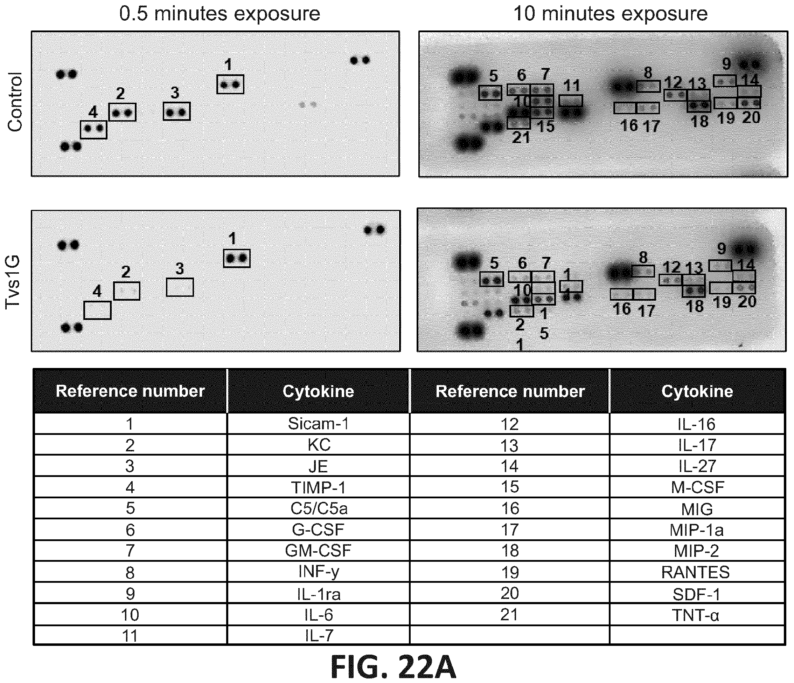

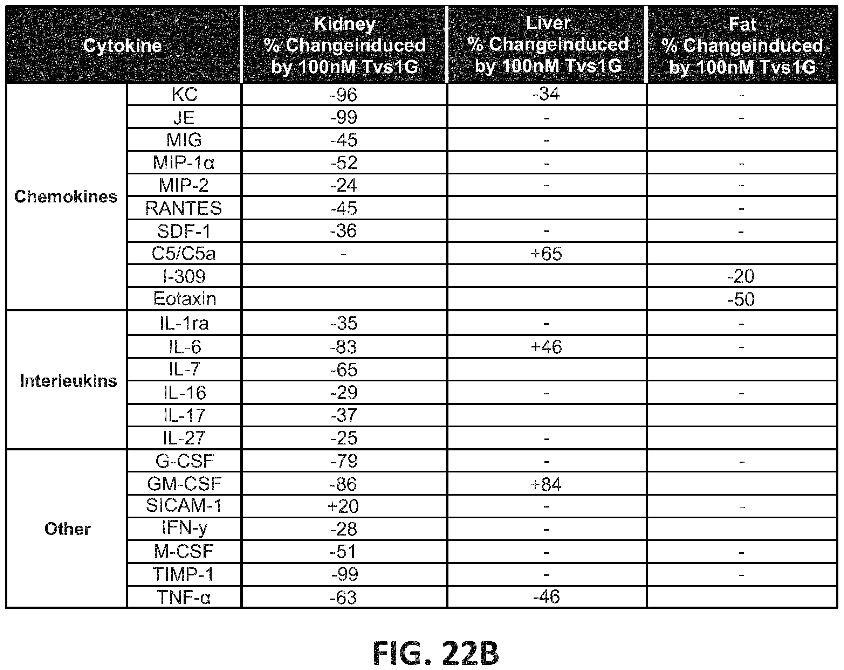

**Figure 22****.** (**A**) Images of arrays for the SASP secreted by kidney from aged mice on a high fat diet treated ex vivo for 48h with 100nM of TVS1G or vehicle (PBS) (B) Relative quantification of the arrays obtained from ex vivo treatments with 100nM Tvs1G in kidney, liver, and adipose tissue. Data are expressed as a change in the percentage of cytokine detection compared to vehicle. Change induced by Tvs1=(Tvs1-treated raw intensity density -vehicle raw intensity density)/ vehicle raw intensity x100.

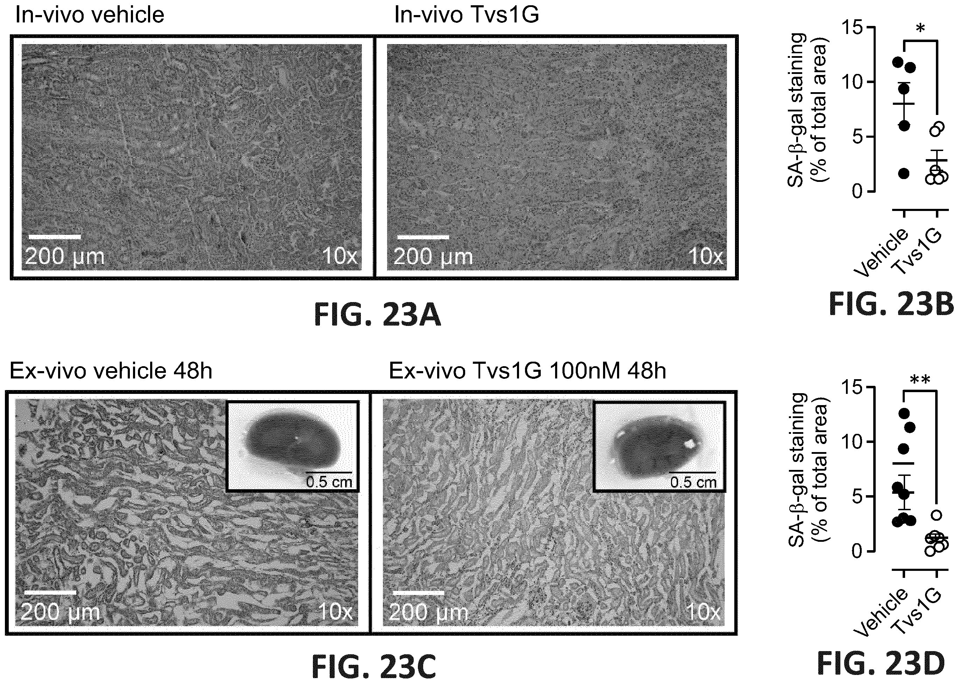

**Figure 23****.** Representative images and quantification of β-galactosidase staining in kidney from old mice on treated (**A-B**) in vivo for one month with vehicle (PBS) or Tvs1G 40 µg/Kg. (C-D) Ex vivo with Tvs1G 100nM for 48h. Data are shown as mean ± SEM. n = 5-7. * p < 0.05 and **p < 0.01 for the indicated comparisons.

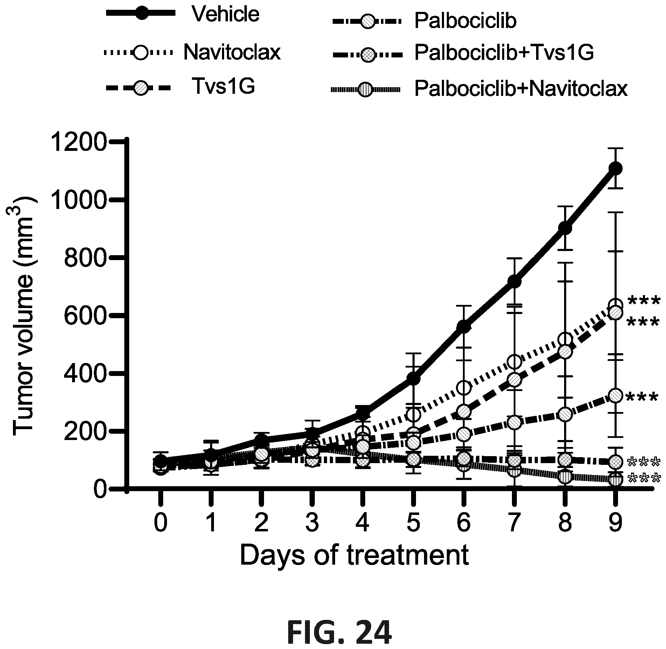

**Figure 24****.** Tumour progression in a xenograft model of senescence induced by palbociclib (100 mg/Kg) evaluated as the fold-change relative to the tumor volume observed prior to initiation of treatments (day 0) with palbociclib+navitoclax (50 mg/Kg, daily, oral gavage, 8 doses) or palbociclib+TVS1G (2 mg/Kg, every 48h, i.p, 4 doses). Xenograft tumours were measured every day using a digital calliper and applying the algorithm: volume = a x b x b x 0.5, where "a" is the length and "b" is the measured breadth of the tumour lump (skin included). Tumour progression was estimated by calculating the fold-change in size versus the initial size prior to the initiation of the treatment (day 0). Data are means±SD, n=4-6 ***, p<0,001 vs vehicle (black) or vs Palbociclib (stripped)

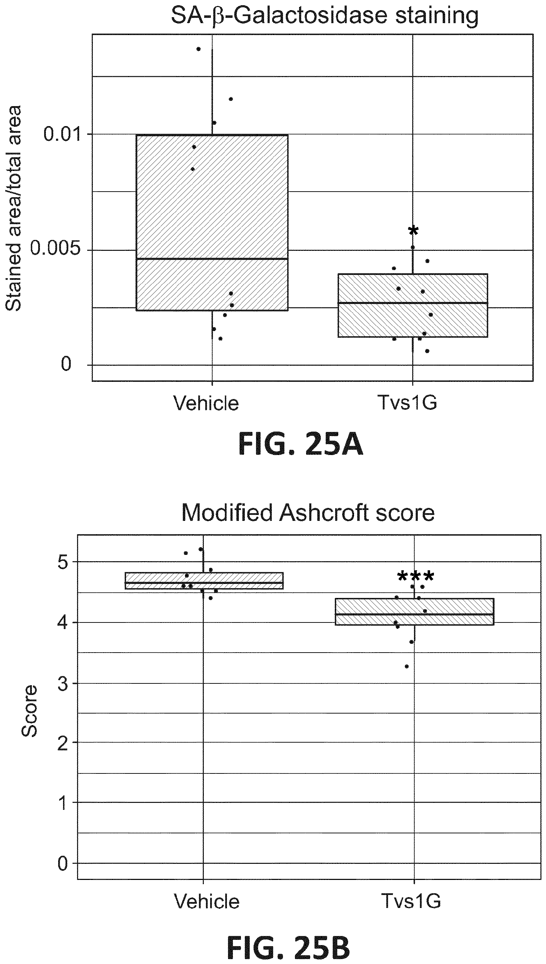

**Figure 25****.** Lung histological analysis of the in vivo effects of Tvs1G 6mg/Kg on (**A**) senescent-associated-β-galactosidase staining and (**B**) Ashcroft score for the severity of lung injury and fibrosis after bleomycin induction. Data are means±SD, n=11, *p<0,05 and ***p < 0.001

### DESCRIPTION OF THE INVENTION

The fields of oncology and age-related diseases are in need for more specific, and less toxic, new-generation of senotherapeutics (senolytic and senomorphic) agents.

In response to the needs of the state of the art, the present invention provides with isolated small proteins, with amino acid sequences SEQ ID NO 1 or SEQ ID NO 2, or fragments thereof, for use as new senolytic and/or senomorphic agents.

The authors of the invention have shown that the marine venom derived protein Sticholysin I (hereinafter Tvs1), from the Caribbean Sea anemone *Stichodactyla helianthus,* with a sequence of 176 aa (SEQ ID NO 1), has senolytic and senomorphic properties. In particular, small protein of sequence SEQ ID NO 1 has a senolytic effect in cancer cells, independently of the stimulus used to induce the cellular senescence.

Tvs1 counts on a therapeutic window. The IC50 of the native Tvs1 differentiates the cytotoxic effect in proliferative cells (IC50=240 nM) and the senolytic effect in senescent cells (IC50=30 nM).

Furthermore, the authors have performed molecular modelling and conducted two main modifications in the C-terminus (6-His) and a methionine insertion at the beginning of the N-terminus in Tvs1 (SEQ ID NO 1), obtaining a new peptide (hereinafter Tvs1G), with sequence of SEQ ID NO 2. These modifications reduce the overall hydropathy index of the protein by changing the grand average of hydropathy score from -0.276 in the native form (SEQ ID NO 1), to -0.36 in the modified version (SEQ ID NO 2). Additionally, Zimmerman and Kyte - Doolittle scales (https://web.expasy.org/protscale/) determined an increased hydrophilicity at the modified C-terminal end of Tvs1G.

The modified protein (Tvs1G) improved the therapeutic window by reducing the toxicity in proliferative cells (IC50=1µM) without affecting the senolytic potency (IC50=30nM). This results in an increase in the senolytic index from 8x inTvs1 to 31x in Tvs1G.

Therefore, a first embodiment of the present invention refers to an isolated small protein consisting of SEQ ID NO 2 and its use as a therapeutic agent, in particular as senolytic and senomorphic agent.

Comparing this protein of SEQ ID NO 2 to other senolytics, this protein is 13 times more potent than navitoclax and with a senolytic index that is 9 times higher. Moreover, the small protein of SEQ ID NO 2 succeeded to consistently induce senolysis where other common senolytics showed to be ineffective.

From the known senolytics, only navitoclax consistently targets senescent cancer cells and could be directly compared with Tvs1 (SEQ ID NO 1) and Tvs1G (SEQ ID NO 2). D+Q/D+F are applicable for senescent fibroblasts while digoxin was not effective in the tested senescent melanoma cells. It was observed that Tvs1G (SEQ ID NO 2) has a broad senolytic spectrum in cancerous lines, where other senolytic consistently failed (Q, F, D, D+Q). Most importantly, SEQ ID NO 2 strongly synergises with a CDK4/6 inhibitor (Palbociclib) for remission of solid tumours, while gross-toxicological studies showed no signs of toxicity (see examples below).

Therefore, a second embodiment of the present invention refers to an isolated small protein having an amino acid sequence selected from SEQ ID NO 1 (Tvs1) or SEQ ID NO 2 (Tvs1G), or fragments thereof, for use as a senolytic and/or senomorphic agent in the prevention or treatment of aging and age-related diseases.

For the purpose of the present invention, the expression "small protein" refers to a compound of less than 20 kilo dalton (kDa) and 200 amino acids in length.

According to the present invention "fragments thereof", refers to parts of the whole sequence of the small protein of the invention (SEQ ID NO 1 or SEQ ID NO 2). These parts can be truncated forms of protein or combined truncated forms belonging to the small protein.

The terms "treatment" or "prevention" as used herein, refer to therapeutic or prophylactic treatment wherein the object is to inhibit, prevent, reduce, slow down or alleviate an undesired physiological change or disorder, such as the progression and/or symptoms of aging and age-related disease. As to the present invention, beneficial or desired clinical results include e.g. alleviation of symptoms, diminishment of extent of disease, stabilized (i.e. not worsening) state of disease, delay or slowing of disease progression, amelioration or palliation of the disease state, and remission (whether partial or total). Subjects in need of treatment or preventive treatment include those already with the disorder as well as those prone to have or develop the disorder e.g. due to genetic predisposition, work or lifestyle choices (e.g., melanoma due to being constantly exposed in the sun without protection) and/or family background.

Examples of aging and age-related diseases, which have been associated with accumulation of SnCs, are (non-exhaustive list): skin disorders, such as wound healing and cosmetic skin aging changes, obesity, obesity-related disease, chronic diseases, progeria syndrome, sarcopenia, fibrotic disease, atherosclerosis, cardiovascular disease, cancer, ostreoarthritis, arthritis, cataracts, osteoporosis, diabetes, Alzheimer's disease, hair loss, hypertension, inflammatory disease, dementia, kidney disease, muscular atrophy, neurological disease, pulmonary disease, vertebral disc degeneration and alopecia.

Aging and many aging-associated diseases are characterised by an increase of cellular damage and persistent accumulation of senescent cells that have a pathological impact on aging-related chronic diseases. Accumulation of senescent cells is a hallmark of aging but occurs also in response to diverse stimuli, including deregulated nutrient sensing, excess feeding and triggered obesity.

Chronic deregulated nutrient sensing disorders and other metabolic diseases such as diabetes lead to the accumulation of senescent cells in major and peripheral tissues (e.g. liver, pancreas, adipose tissue, kidney). Such is the case of obese people with sedentary habits that have excessive ongoing caloric intake or exhibiting diminished energy expenditure. It has been further shown that obese people exhibit increased SAβGAL activity- and SASP production in the adipose tissue as well as elevated adipose-p53 and p21 levels, which contributes to the disease pathology.

Fibrosis and wound healing are intricately linked processes that occur in response to tissue injury. This complex cascade involves a series of events, including injury initiation, inflammation, proliferation and migration of fibroblasts, and deposition and remodelling of the extracellular matrix. This is particularly pronounced in the case of idiopathic pulmonary fibrosis (IPF), a disease commonly associated with aging *(*Barnes, P. J., et al. Cellular Senescence as a Mechanism and Target in Chronic Lung Diseases. Am J Respir Crit Care Med, (2019) 200(5), 556-564*;* Tzouvelekis, A., et al. Common Pathogenic Mechanisms Between Idiopathic Pulmonary Fibrosis and Lung Cancer. Chest, (2019) 156(2), 383-391*).* Lung decline in IPF is primarily due to the destruction of lung parenchyma, which is characterized by distinctive alveoli obliteration and accumulation of fibroblastic masses. There is compelling information suggesting that cellular senescence plays a pivotal role in the pathogenesis of lung fibrosis *(*Aoshiba, K., et al. Senescence-associated secretory phenotype in a mouse model of bleomycin-induced lung injury. Experimental and Toxicologic Pathology, (2013), 65(7), 1053-1062*;* Faner, R.,et al. Abnormal Lung Aging in Chronic Obstructive Pulmonary Disease and Idiopathic Pulmonary Fibrosis. American Journal of Respiratory and Critical Care Medicine, (2012) 186(4), 306-313*;* Schafer, M. J., et al. Cellular senescence mediates fibrotic pulmonary disease. Nature Communications (2017) 8(1), Article 1*).* Senescent cells contribute to chronic inflammation, fibroblast activation, and impaired tissue regeneration, all of which promote fibrosis development and progression *(Barnes et al., 2019; Tzouvelekis et al., 2019).* Understanding the role of cellular senescence in lung fibrosis has opened new avenues for therapeutic interventions. Targeting senescent cells, either through selective elimination or modulation of their senescence-associated secretory phenotype, has shown promising results in animal models *(Lim et al., 2015; Schafer et al., 2017; Triana-Martinez et al., 2019)* and humans *(Nambiar et al., 2023).*

In a preferred embodiment the age-related diseases are at least one of chronic disease, such as cancer, fibrosis disease, e.g., lung fibrosis, aged-related obesity, kidney disease, and progeria syndrome. Cancer disease is preferable referred to melanoma, hepatoma, lung adenocarcinoma and neuroblastoma.

In particular embodiments, the isolated small proteins of the invention reduce solid tumour progression, alone or in combination with other active compounds.

In another embodiment, the present invention refers to a pharmaceutical composition comprising the small protein of the invention and, optionally, a pharmaceutically acceptable excipients or carrier, wherein the protein is present in a therapeutically effective amount. In a preferred embodiment, the proteins or the pharmaceutical compositions are administered orally or by means of an intraarticular, subcutaneous, intraperitoneal, intravenous or muscular injection.

For the purpose of the present invention, the expression *"therapeutically effective amount*" refers to the situation when the small proteins are administered and brings about a positive therapeutic response in a subject having an age-associated disease in comparison to the vehicle-control. The exact amount required will vary from subject to subject, depending on the species, age, and general condition of the subject, the severity of the condition being treated, mode of administration, duration, experimental design etc. An appropriate "effective" amount in any individual case may be determined by one of ordinary skill in the art using routine experimentation, based upon the information provided herein.

According to the present invention "pharmaceutically acceptable excipient or carrier" refers to an excipient that may optionally be included in the pharmaceutical composition of the invention that causes no significant adverse toxicological effects to the patient.

The proteins of the invention can be administered with one or more additional therapeutic agent, either separately or in the same formulation as the protein of the invention.

Therefore, in a particular embodiment, the pharmaceutical composition of the invention further encompasses at least one further active compound. In one particular embodiment, the further active compound is selected from one or more of a CDK4/6 inhibitor (e.g., Palbociclib, ribociclib, abemaciclib), a genotoxic chemotherapy agent (e.g. doxoroubicin, etoposide, daunorubicin, mitoxantrone, irinotecan, paclitaxel), non-genotoxic chemotherapy agent (e.g. nutlins, tamoxifen, fulvestrant, dasatinib, vemurafenib, sorafenib) radioactivity (external beam, brachytherapy or systemic therapy) or after multiple cell passages. In another embodiment, the pharmaceutical composition is further combined with a cardiac glycoside (e.g. digoxin), that acts synergistically with the protein of the invention.

Accumulation of senescent cells with age also contribute to age-related skin changes and pathologies. By affecting molecular pathways of senescence, the small proteins of the invention are able to avoid or ameliorate aging and age-associated pathologies of the skin.

Therefore, in another embodiment, the present invention refers to a cosmetic composition comprising the small protein of the invention for use in skin condition improvement, such as skin regeneration, skin elasticity improvement, skin wrinkle prevention or improvement, skin aging prevention or improvement, skin inflammation improvement and skin whitening improvement.

The cosmetic composition of the present invention may be formulated in the form of a liquid, ointment, cream, lotion, spray, patch, gel or aerosol and may further include conventional adjuvants and carriers such as antioxidants, stabilizers, solubilizers, vitamins, pigments, fragrances, and other special ingredients used in cosmetic formulations (usually natural-based) to act synergistically with the compounds of the invention.

As it is shown in the results provided by the present invention, mechanistically, it has been identified that the small proteins of the invention (SEQ ID NO 1 and SEQ ID NO 2):
- depolarize mitochondrial membrane potential and reduce mitochondrial respiration in senescent cells, but not in proliferative cells,
- reduce the senescence-associated secretory phenotype (SASP) at low concentrations in senescent SKMel103 cells,
- facilitate ionic imbalance with a potassium loss and membrane hyperpolarization of senescent cells,
- affect the potency of the small proteins of the invention. Data indicates that these small proteins bind preferentially to POPC-enriched lipid cell membranes, while an elevated content of sphingomyelin reduces the effects of these proteins in senescent cells, and
- reduce the content of proinflammatory lysophospholipids (lysophosphatidylinositol, LPI; and lysophosphatidylserine, LPS) specifically in senescent cells.

These results support that senescent cells have a distinct lipid profile in comparison to their proliferative counterparts. Specifically senescent cells have higher levels of LPI and LPS. The proteins of the invention do not alter lysophospholipids concentrations in proliferative cells but in senescent cells, diminishing specifically LPI and LPS cellular content, which modulate cell growth and metabolism. These lipids are of particular interest as lysophospholipids are multifunctional mediators in inflammation and paracrine responses and their reduction is relevant for SASP modulation. Thus, results and examples provided by the invention show that LPS and LPI constitute a target of the compounds of the invention in senescent cells.

Attending to the binding specificity of these compounds to lipid membranes another aspect of the invention refers to a method for identifying a compound for the treatment or prevention of aging and age-related diseases comprising: contacting a senescent cell with a test compound and determining if the test compound reduces the lysolipids, LPS and LPI, cellular content; thereby identifying an effective compound (with senolytic and/or senomorphic activity) for the treatment or prevention of aging and age-related diseases. The test compound can be selected from compounds of different natural, recombinant or chemical origin such as small protein (purified or produced), a peptide, a polypeptide, a nucleic acid molecule, small compound, antibody, etc.

### EXAMPLES

Specific embodiments of the invention that serve to illustrate the invention without limiting the scope thereof are described in detail below.

### Material and methods

### Reagents and media

Media for cell culture was purchased from ThermoFisher Scientific (Waltham, MA USA). Drugs were purchase from MedChemTronica (Sollentuna, Sweden) and Sigma Aldrich (Merck KGaA, Darmstadt, Germany).

### Synthesis of Tvs1 and analogues

Tvs1 (SEQ ID NO 1), Tvs2 (SEQ ID NO 3) and Tvs1 E2AD9A (SEQ ID NO 4) were provided by Alvaro Martinez del Pozo (University Complutense of Madrid) and produced as previously described in Rivera-De-torre, E., et al. Functional and structural variation among sticholysins, pore-forming proteins from the sea anemone stichodactyla helianthus. International Journal of Molecular Sciences, (2020) 21(23), 1-24)*.*

Later modifications of Tvs1 included inclusion of methionine at the N-terminal end and six residues of histidine at the C-terminus. This new version is referred as Tvs1G throughout the specification (SEQ ID NO 2). Tvs1G was recombinantly produce with pET30a-Tvs1G (cloned in Ndel-Hindlll) and purified in Ni²⁺-NTA columns by GenScript (Leiden, The Netherlands). Endotoxin removal was applied to archived maximum values between 0.1-1 Eu/mg. Purity was tested by SDS-PAGE under reducing condition. The product was stored at -80 °C in 50 mM Tris-HCl, 150 mM NaCl, 10% Glycerol, pH 8.0.

### Cell culture

All human cancer cell lines and the non-transformed fibroblast lines were maintained in a humidified incubator at 37°C and 5% CO₂ and used between passages 4 and 15. All lines were frequently checked and ensured to be mycoplasma free. Cells were split twice a week and before archiving an 90% confluency. SKMel103, SH-SY5Y, Huh7, A549, U251 were donated from collaborators or obtained from the Translational Venomics' stock. Primary lung fibroblasts (PCS201013) and primary dermal fibroblasts (PCS201012) were purchased from ATCC (Manassas, VA, USA). Skin fibroblasts from Hutchinson-Gilford progeria syndrome donors were purchase from the Progeria Research Foundation (HGADFN 127, 143, 167, 178) or Coriell Institute (AG01972E, AG06917K and AG11513F). The cells were maintained following the recommendations of the cell banks.

### Cell viability assay

Cells were plated in 96 well plates at a density of 3×10³-8×10³ cells/well (proliferative) or 8×10³-10×10³ cells/well (senescent). An MTT assay (Sigma-Aldrich, Merck KGaA, Darmstadt, Germany) was used to measure cell viability after 48-96 h of treatments. Briefly, MTT was diluted in 0.1 M phosphate buffered saline (PBS) and added to cells grown in 96-well plates at a final concentration of 0.5 mg/ml. After incubation for 2-4 hr at 37°C with MTT, the culture medium was removed. The precipitated formazan was solubilized with dimethyl sulfoxide (DMSO). Absorbance was measured at 570 nm using a microplate reader (Victor Nivo; Perkin Elmer, Hamburg, Germany). For these assays, the effects of vehicle or treatments were expressed as the % of change of the non-treated cells.

### Cell cycle analysis

Cell cycle phases were determined using propidium iodide (PI) stain. In brief, 10⁵ Cells were harvested and fixed drop wise in cold ethanol 70%. Fixed cells were maintained at -20°C over night. Cells were then centrifuged and washed with PBS. Samples were treated with 100 µg/mL RNAse A, 50 µg/mL PI and incubated in the dark for at least 30 min. Mean fluorescence intensity from 10,000 cells was acquired by FACScelesta flow cytometer (BD Bioscience, New Jersey, USA) using the 610/20 bandpass filters.

### Apoptosis assay

The annexin V-FITC/PI apoptosis detection kit (BD Biosciences, New Jersey, USA) was used to detect different stages of apoptosis. Annexin V has a strong affinity for phosphatidyl serine, which is externalized in the membranes of apoptotic cells. In brief, cells were exposed to treatments or vehicles for 48 hr, washed in PBS, and resuspended in binding buffer (HEPES-NaOH 10 mM pH 7.4, 144 mM NaCl and 25 mM CaCl2). Annexin V-FITC (0.2 µg/µl) and propidium iodide (PI, 0.05 µg/µl) were added, and the cells were incubated in the dark for 20 min. Mean fluorescence intensity from 10,000 cells was acquired by FACSCelesta flow cytometer (BD Bioscience, New Jersey, USA) using the FITC and PE bandpass filter for annexin V and PI, respectively.

### Reactive Oxygen Species assay

ROS levels were monitored using carboxy-2',7'-dichlorodihydro-fluorescein diacetate (carboxy-H2DCFDA, Molecular Probes-Invitrogen, Carlsbad, CA, USA). After the indicated treatments, cells were washed with PBS; incubated with carboxy-H2DCFDA (10 µM, 45 min, 37°C) in media; washed twice with PBS; scraped into a 5 ml tube and analyzed by flow cytometry. Mean fluorescence intensity from 10,000 cells was acquired by FACSCelesta flow cytometer (BD Bioscience, New Jersey, USA) using a FITC bandpass filter, and expressed as percentage of the control.

### Mitochondrial Membrane Potential

Mitochondrial membrane potential was determined using rhodamine 123 (ThermoFisher Scientific, Waltham, MA USA) and TMRM (ThermoFisher Scientific ,Waltham, MA USA). After the indicated treatments, cells were washed with PBS; incubated with rhodamine 123 (10 µg/mL, 45 min, 37°C) or TMRM (100nM) in media; washed twice with PBS; scraped into a 5 ml tube and analyzed by flow cytometry. Mean fluorescence intensity from 10,000 cells was acquired by FACScelesta flow cytometer (BD Bioscience, New Jersey, USA) using a FITC or PE bandpass filters for detection of rhodamine123 or TMRM, respectively.

### Bioenergetics

Cellular bioenergetic measurements were performed using the Seahorse XFe96 Analyzer and XFe96 culture microplates (AgilentTechnologies, Madrid, Spain) to investigate Oxygen Consumption Rates (OCR) and Extracellular Acidification Rates (ECAR) in proliferative or senescent SKMel103 cells. Measurements were performed after 2-24 h incubation with 100 nM Tvs1, TVS1G or analogue (Tvs2) in 25×10³ proliferative or senescent SKMel cells/well in quadruplicates. OCR was tested in Seahorse XF base medium containing 10 mM glucose, 1 mM sodium pyruvate, and 2 mM L-glutamine (pH 7.4) and ECAR in Seahorse XF base medium containing 0.5 mM sodium pyruvate and 1 mM L-glutamine (pH 7.4). Data were expressed as percentage of the basal respiration per well.

### Electrophysiological studies

Membrane currents were recorded with an Axopatch 200B and a Digidata 1322A (Axon Instruments, Burlingame, CA, USA) using the whole-cell configuration of the patch-clamp technique. Cells were superfused at 1 ml min⁻¹ with bath solution containing (in mM) NaCl 130, KCI 5, CaCl₂ 1.8, MgCl₂ 1.2, glucose 10, and HEPES 10 (pH 7.3 with NaOH) and a pipette (internal) solution containing (in mM) KCI 135, MgCl₂ 1.2, Na₂ATP 5, HEPES 10, and EGTA 0.1 (pH adjusted to 7.2 with KOH). Cells were clamped at -60mV and potassium currents were assessed by voltage ramps (-100 to +60 mV), single voltage steps (-100 and +60 mV) and by acquisition of full I-V relationships for steady state activation (4 s steps from -100 to +60 mV in 10 mV increments). Current magnitude was normalised to cell capacitance as required. Membrane potential was recorded under the current-clamp mode. Signals were sampled at 10 kHz and low-pass filtered at 2 kHz. Voltage-clamp acquisition and analysis protocols were controlled by Clampex 10.0 software (Molecular Devices, Sunnyvale, CA, USA). Off-line analysis was performed using Clampfit 10.0 (Molecular Devices). Data are expressed as current density (pA/pF) or I/Izero, where Izero is the current magnitude recorded at the onset of a given experimental intervention. All experiments were performed at room temperature (22-24°C).

### Cytokine arrays

Proliferative or senescent (palbociclib 5µM, 7days) SKMel-103 cells were plated in a 6 well plate at a confluency of 50×10³ cell/well (proliferative) or 300×10³ cell/well (senescent). Senescent cells were then treated in triplicates with vehicle (0.1% PBS), Tvs1 10-30 nM for 4 days in complete DMEM (10% FBS, 1% penicillin/streptomycin, 1% non-essential aminoacids). Proliferative SKMel103 were treated only with vehicle (PBS 0.1%) as a parallel control. After 4 days, all groups were trypsinized, viable cells were counted using the trypan blue exclusion method and plated at a confluency of 500×10³ cell/well (duplicate per group, 6 well plate) in 1.5 mL of conditioned media (DMEM 0% FBS, 1% penicillin/streptomycin, 1% non-essential aminoacids) for24h. Cytokine levels in conditioned media were analyzed using the Proteome Profiler Human Cytokine Array (R&D Systems; #ARY005B), following the manufacturer's instructions. Pixel density was determined using the ImageStudio Software.

In the progeria data, HGADFN 127 skin fibroblast cells were plated in a 6 well plate at a confluency of 400×10³ cell/well. Fibroblasts were then treated in triplicates with vehicle (0.1% PBS) or Tvs1G 100nM for 24h in complete DMEM (10% FBS, 1% penicillin/streptomycin, 1% non-essential aminoacids). After 1 day, both groups were trypsinized, viable cells were counted using the trypan blue exclusion method and plated at a confluency of 450×10³ cell/well (duplicate per group, 6 well plate) in 1.5 mL of conditioned media (DMEM 0% FBS, 1% penicillin/streptomycin, 1% non-essential aminoacids) for 24h. Cytokine levels in conditioned media were analyzed using the Proteome Profiler Human Cytokine Array (R&D Systems; #ARY005B), following the manufacturer's instructions. Pixel density was determined using the autoradiography and imaged.

For serum and ex-vivo kidney studies in mouse tissue, treatments were performed with TVS1G. Cytokine levels in conditioned media were analyzed using the Proteome Profiler Array (R&D Systems; mouse cytokine array panel A, #ARY006), following the manufacturer's instructions. Pixel density was determined using the ImageStudio Software.

### Ex-vivo assays

In these assays, liver, kidney and white adipose tissue were isolated from old female mice (19-22 months old) previously fed on high-fat diet for 4 months as described in the animal studies methods. Tissue was cut into similar pieces, weighted, washed with PBS 3 times and cultured in DMEM containing, 1% MEM non-essential amino acids, 5% penicillin and streptomycin (15140; Thermo Fisher Scientific) 1% amphotericin B (A2942; MilliporeSigma, Burlington, MA, USA) with vehicle, 100 nM Tvs1G or 1 µM navitoclax. After 48 h, the tissue explants were washed 3 times with PBS and was then maintained in media without drugs for 24 h to collect conditioned medium for the mouse cytokine array panels. The volume of media was adjusted according to the weight of the tissue to maintain a ratio of 0.2 g/mL throughout the entire procedure. The tissue explants then were fixed and stained to detect senescence-associated β-galactosidase activity.

### Senescence-associated β-galactosidase (SA-βGal) staining

For the evaluation of the SA-βGal staining, 50×10³-80 ×10³cells were seeded in coverslips (22x22 mm) and left overnight in complete media. The following day, media was removed, and the cells were washed, fixed and stained overnight following the manufacturer's instructions for the Cell Signaling SA-βGal staining Kit (#9860, Cell Signaling Technology, Massachusetts, USA).

For whole-tissue SA-βGal staining, frozen tissue was fixed at room temperature for 1 hour in 2% formaldehyde / 0.2% glutaraldehyde, washed twice (PBS, 2 mM MgCl₂ pH 6.0) and incubated under continuous agitation with SA-βGal solution (20 mg/ml X-Gal in dimethylformamide; 100 mM K₄[Fe(CN)₆]; 100 mM K₃[Fe(CN)₆]; 150 mM NaCl in PBS; 2 mM MgCl₂ pH 6.0) for 24h at 37°C. After that time, samples were washed twice with PBS and dehydrated with increasing EtOH concentrations (30% for 15 minutes, and then 70% for 30 minutes). Samples were then kept in 70% EtOH until embedded in paraffin, sectioned and counter-stained with nuclear fast red for microscope image acquisition. Analysis of SA-βGal staining were performed from 4 fields (10x) per animal using the imaged software (National Institutes of Health, Tennessee, USA) and expressed as a ratio between the stained area and the total area.

### RNA Sequencing

Senescent SKMel103 cells (570×10³ cell/well) were treated with Tvs1 100nM following a time course at 0, 3,6 and 12 h. The cells were then collected, washed with PBS, and RNA extraction was performed within 2h post lysis using Qiagen RNeasy kit (QIAGEN, Venlo, The Netherlands) according to manufacturer's directions. RNA integrity was checked in 2% agarose gels. RNA purity and concentration was assessed using NanoDrop 2000 (Thermo Fisher Scientific, Waltham, MA USA). The RNA samples were sent for sequencing to Biomarker Technologies (BMKGENE, Beijing, China)

Library preparation for Transcriptome sequencing. A total amount of 1 µg RNA per sample was used as input material for the RNA sample preparations. Sequencing libraries were generated using Hieff NGS Ultima Dual-mode mRNA Library Prep Kit for Illumina (Yeasen Biotechnology, Shanghai) Co., Ltd.) following manufacturer's recommendations and index codes were added to attribute sequences to each sample. Briefly, mRNA was purified from total RNA using poly-T oligo-attached magnetic beads. First strand cDNA was synthesized, and second strand cDNA synthesis was subsequently performed. Remaining overhangs were converted into blunt ends via exonuclease/polymerase activities. After adenylation of 3' ends of DNA fragments, NEBNext Adaptor with hairpin loop structure were ligated to prepare for hybridization. The library fragments were purified with AMPure XP system (Beckman Coulter, Beverly, USA). Then 3 µl USER Enzyme (NEB, USA) was used with size-selected, adaptor-ligated cDNA at 37°C for 15 min followed by 5 min at 95°C before PCR. Then PCR was performed with Phusion High-Fidelity DNA polymerase, Universal PCR primers and Index Primer. At last, PCR products were purified (AMPureXP system) and library quality was assessed on the Agilent Bioanalyzer 2100 system.

Sequencing. The libraries were sequenced on an Illumina NovaSeq platform to generate 150 bp paired-end reads, according to the manufacturer's instructions.

### Lipid extraction for mass spectrometry lipidomics

It was performed lipid extraction for proliferative and senescent SKMEL103 cells induced by Palbociclib (5µM, 7 days). In brief, four 75cm³-flask containing in 1.8 million cells were treated for 12h with 100nM of TVS1G or vehicle. Mass spectrometry-based lipid analysis was performed by Lipotype GmbH (Dresden, Germany) as described (S. Surma, M. et al. Lipids Health Dis (2021) Vol. 20 Issue 1 Pages 141*).* Lipids were extracted using a chloroform/methanol procedure (C. S. Ejsing, J. L. et al. Global analysis of the yeast lipidome by quantitative shotgun mass spectrometry. Proc Natl Acad Sci USA (2009) Vol. 106 Issue 7 Pages 2136-41*).* Samples were spiked with an internal lipid standard mixture containing: cardiolipin 14:0/14:0/14:0/14:0 (CL), ceramide 18:1;2/17:0 (Cer), diacylglycerol 17:0/17:0 (DAG), hexosylceramide 18:1;2/12:0 (HexCer), lyso-phosphatidate 17:0 (LPA), lyso-phosphatidylcholine 12:0 (LPC), lyso-phosphatidylethanolamine 17:1 (LPE), lyso-phosphatidylglycerol 17:1 (LPG), lyso-phosphatidylinositol 17:1 (LPI), lyso-phosphatidylserine 17:1 (LPS), phosphatidate 17:0/17:0 (PA), phosphatidylcholine 17:0/17:0 (PC), phosphatidylethanolamine 17:0/17:0 (PE), phosphatidylglycerol 17:0/17:0 (PG), phosphatidylinositol 16:0/16:0 (PI), phosphatidylserine 17:0/17:0 (PS), cholesterol ester 16:0 D7 (CE), sphingomyelin 18:1;2/12:0;0 (SM), triacylglycerol 17:0/17:0/17:0 (TAG). After extraction, the organic phase was transferred to an infusion plate and dried in a speed vacuum concentrator. The dry extract was re-suspended in 7.5 mM ammonium formiate in chloroform/methanol/propanol (1:2:4; V:V:V). All liquid handling steps were performed using Hamilton Robotics STARlet robotic platform with the Anti Droplet Control feature for organic solvents pipetting.

Samples were analyzed by direct infusion on a QExactive mass spectrometer (Thermo Scientific) equipped with a TriVersa NanoMate ion source (Advion Biosciences). Samples were analyzed in both positive and negative ion modes with a resolution of R_{m/z}=₂₀₀=280000 for MS and R_{m/z}=₂₀₀=17500 for MSMS experiments, in a single acquisition. MSMS was triggered by an inclusion list encompassing corresponding MS mass ranges scanned in 1 Da increments (Surma et al. 2015). Both MS and MSMS data were combined to monitor CE, DAG and TAG ions as ammonium adducts; LPC, LPC O-, PC and PC O- as formiate adducts; and CL, LPS, PA, PE, PE O-, PG, PI and PS as deprotonated anions. MS only was used to monitor LPA, LPE, LPE O-, LPG and LPI as deprotonated anions, and Cer, HexCer and SM as formiate adducts.

Data were analyzed with in-house developed lipid identification software based on LipidXplorer (R. Herzog, D., et al. Genome Biol 2011 Vol. 12 Issue 1 Pages R8. Accession Number: 21247462 PMCID: PMC3091306 DOI: 10.1186/gb-2011-12-1-r8*;* R. Herzog, K. Schuhmann, D. Schwudke, J. L. Sampaio, S. R. Bornstein, M. Schroeder, et al. PLoS One 2012 Vol. 7 Issue 1 Pages e29851. Accession Number: 22272252 PMCID: PMC3260173 DOI: 10.1371/journal.pone.0029851). Data post-processing and normalization were performed using an in-house developed data management system. Only lipid identifications with a signal-to-noise ratio >5, and a signal intensity 5-fold higher than in corresponding blank samples were considered for further data analysis.

### Animal studies

### Xenograft model

Mouse experiments were performed under the Institute for Research in Biomedicine (IRB, Barcelona, Spain) ethical guidelines and regulations. The experimental protocols described were approved by the IRB Animal Ethics Committee (Approval Number 23-033-PIL). BALB/c FoxN1 nude mice were purchased from Envigo Spain and were housed in strict animal husbandry facilities free of pathogens and micro-organisms. The facilities had controlled temperature environment and at normal 12h day-night light cycles. Mice were randomly selected for the peptide or vehicle treatment and placed in four cages with 4 animals each. Each cage had bedding to provide shelter and privacy as well as unlimited access to food and water. Animals were daily inspected by the qualified personnel and weekly or as needed by the institute's veterinary doctor.

First a pilot study was conducted to determine the maximum safe dose of Tvs1G to inject into mice. For this experiment, four mice initially received an intraperitoneal (i.p.) injection of Tvs1G 40ug/kg in PBS; every other day the dose was doubled in order to determine the maximum tolerated dose. Mice were closely monitored every day to check for possible adverse effects. The maximal safe dose to administer was 2mg/kg. Eight weeks old female BALB/c FoxN1 nude mice were injected subcutaneously (s.c.) with 10⁶ SKMel103 cells in both flanks to develop two tumours per mouse. When the tumours reached a size of approximately 75 to 100mm³, the mice were randomly divided in 5 groups of 4 mice each: untreated, palbociclib, Tvs1G, palbociclib + Tvs1G and palbociclib + navitoclax (as a senolytic control).

Palbociclib (100mg/kg) was administered by oral gavage every day for 10 days; palbociclib treatment was started two days before initiating the other treatments. Tvs1G (2 mg/kg) or vehicle (PBS) were administered i.p. every two days for a total of 8 days. Navitoclax (50mg/kg) was administered by oral gavage every day for a total of 8 days. Xenograft tumours were measured every day using a digital calliper and applying the algorithm: volume = a x b x b x 0.5, where "a" is the length and "b" is the measured breadth of the tumour lump (skin included). Tumour progression was estimated by calculating the fold-change in size versus the initial size prior to the initiation of the treatment (day 0). At the end of the experiment, mice were euthanised by CO₂ and tumours were harvested for histology and protein/RNA extraction. From each mouse kidneys, liver, lung, heart, brain and blood were isolated, fixed (4% PFA) or stored at - 80°C for further analysis.

### Lung fibrosis model and evaluation of lung fibrosis

Mouse experiments were performed under the Czech Centre for Phenogenomics (CCP, Vestec, Czech Republic) ethical guidelines and regulations. The experimental protocols described were approved by the CCP Animal Ethics Committee. Animals were housed in strict animal husbandry facilities free of pathogens and micro-organisms. The facilities had controlled temperature environment and at normal 12h day-night light cycles. Each cage had bedding to provide shelter and privacy as well as unlimited access to food and water. Animals were daily inspected by the qualified personnel and weekly or as needed by the institute's veterinary doctor. First a first pilot study was conducted to assess maximal safe dose of Tvs1G in this model. For that, 3 male C57BL6 mice of were induced lung fibrosis with acute intratracheal delivery of 2 U/Kg bleomycin on day 1. On day 22 after bleomycin delivery, one mouse was i.p. injected with increasing doses of Tvs1G (6, 8 and 10 mg/Kg) every two days under close monitorization. In 2 additional mice, Tvs1G was i.p injected at a dose of 6 mg/Kg every 2 days for a total of 8 days (4 injections). It was determined that 6 mg/Kg Tvs1G was safe in this model for the studied period of time. On day 23 after bleomycin delivery, 15 C57BL6 mice (6-7 weeks old) were randomly divided into treated group (Tvs1G 6 mg/Kg) or control group (NaCl, 10% glycerol, 50 mM Tris-HCl, pH 8.0 diluted with saline in the same proportion as protein was diluted). Compound and vehicle administration were i.p. injected 4 times every second day for a total of 8 days (4 injections). Animal health monitoring and weighting were done every day during the course of the experiment. Organ fixation collection was done on day 30 after bleomycin delivery.

Lung fibrosis was evaluated using the modified Ashcroft scale for lung histopathology as described in Hübner, R.-H., et al (2008). Standardized quantification of pulmonary fibrosis in histological samples. BioTechniques, 44(4), 507-517. https://doi.org/10.2144/000112729*.* In brief, lung sections stained with Hematoxylin-Eosin were assessed by system of grades (0-8). Slide is evaluated in a raster-like pattern using 20-fold objective and 15 fields per mouse were scored.

| **Score** | **Description** |
|---|---|
| 0 | Alveolar septa: No fibrotic burden at the flimsiest small fibers in some alveolar walls |
| | Lung structure: Normal lung |
| 1 | Alveolar septa: Isolated gentle fibrotic changes (septum ≤3× thicker than normal) |
| | Lung structure: Alveoli partly enlarged and rarefied, but no fibrotic masses present |
| 2 | Alveolar septa: Clearly fibrotic changes (septum >3× thicker than normal) with knot-like formation but not connected to each other. |
| | Lung structure: Alveoli partly enlarged and rarefied, but no fibrotic masses. |
| 3 | Alveolar septa: Contiguous fibrotic walls (septum >3× thicker than normal) predominantly in whole microscopic field. |
| | Lung structure: Alveoli partly enlarged and rarefied, but no fibrotic masses. |
| 4 | Alveolar septa: Variable. |
| | Lung structure: Single fibrotic masses (≤10% of microscopic field). |
| 5 | Alveolar septa: Variable. |
| | Lung structure: Confluent fibrotic masses (>10% and ≤50% of microscopic field). Lung structure severely damaged but still preserved. |
| 6 | Alveolar septa: Variable, mostly not existent. |
| | Lung structure: Large contiguous fibrotic masses (>50% of microscopic field). |
| | Lung architecture mostly not preserved. |
| 7 | Alveolar septa: Non-existent |
| | Lung structure: Alveoli nearly obliterated with fibrous masses but still up to five air bubbles. |
| 8 | Alveolar septa: Non-existent |
| | Lung structure: Microscopic field with complete obliteration with fibrotic masses |

### Diet-induced obesity model in old mice

Experimental research in mice was conducted in accordance with relevant guidelines and regulations of the animal care and use committee of the Centro Nacional de Biotecnologia (CNB, Madrid, Spain), and the Spanish Major Council for Scientific Research (CSIC), which adopts "The European Code of Conduct for Research Integrity" and "Good Experimental Practices". Likewise, the experimental protocol with PROEX reference number 191/18 and 114/19 was approved by the Ethical Committee of the CNB and CSIC. Briefly, 13 C57BL6 female mice 15-17 months of age were fed on a high-fat diet (HFD, Envigo, TD.07734; Protein: 18.8% kCal, Carbohydrate: 41.4% kCal, Fat: 39.7% kCal; https://www.inotivco.com/diet-induced-obesity-custom-diets for four months to develop diet-induced obesity. During this time, body weight was recorded every week. After 4 months of HFD, 19-22 months old mice were randomly allocated into experimental two experimental groups. Group Control (6 mice) was given 0.3 mL PBS i.p., three times a week for 1 month while TVS1 group (7 mice) was given Tvs1G 40 µg/Kg i.p., in PBS three times a week for 1 month. Animals were daily inspected by the qualified personnel within the Hepatic Regenerative Medicine Laboratory at IMDEA Food. At the end of the experiments, mice were anesthetised using isofluorane 2.5 % to harvest blood by cardiac punction and later being euthanised by isofluorane overdose to collect major organs and tissues for further analysis.

### Data analysis

The declared group size is the number of independent values, and statistical analysis was done using these independent values. The effects of Tvs1G were not predictable in vivo, hence, a sample size estimation was not performed, and experiments were evaluated to comply with the 3R principles (replace, reduce, and refine).

In addition, statistical analysis was undertaken for studies where each group size was at least n=3. All data represent a minimum of three independent experiments, were performed in triplicate and are expressed as the mean ± standard deviation (SD) or mean ± standard error of the mean (SEM). Statistical analyses were conducted using either a Student's t-test if only two variables were tested (e.g., control and Tvs1G treated samples) or one-way ANOVA followed by a Sidak test for multiple comparisons. Statistical significance was considered at *P<0.05. **P<0.01 and ***P<0.0001. Of note, in multigroup studies with parametric variables, post hoc tests were conducted only if F in ANOVA (or equivalent) achieved the necessary level of statistical significance (P<0.05) and there was no significant variance in homogeneity. All sample populations were first checked for normality. When populations within groups were not normally distributed, a non-parametric Mann-Whitney test was used for single comparisons or Kruskal-Wallis test for multiple comparisons. For mouse in vivo studies, the permutation test was used to compare tumour progression during the treatments. IC50 values were calculated using a non-linear regression with the model log(inhibitor) vs response and least squares fit. *P<0.05 was considered statistically significant. To control for unwanted sources of variation between individual experiments, data were normalized to the mean of control as indicated in the figure legends. All data were analysed using GraphPad Prism 9 (GraphPad Software, Boston, MA, USA).

### Example 1. In vitro validation of a venom-derived protein from a marine source with senolytic properties.

Senescent cells accumulate in aging tissues and contribute to age-related diseases. Senolytic drugs selectively target and eliminate senescent cells, with the goal of improving healthspan and delaying or preventing age-related diseases and the accompanied ill phenotypes (*Chaib et al., 2022; Kirkland* & *Tchkonia, 2020;* Muñoz-Espin, D. et al. Cellular senescence: From physiology to pathology. Nature Reviews. Molecular Cell Biology, (2014) 15(7), 482-496*).* The MTT assay is a commonly used colorimetric assay that measures cell viability and can be used to evaluate the efficacy of senolytic drugs.

Therefore, the MTT assay was used to measure cell viability in proliferative and senescent cells. The model of cellular senescence induced by cyclin-dependent kinases (CDK) inhibition with Palbociclib (Baughn, L. B., et al. A Novel Orally Active Small Molecule Potently Induces G1 Arrest in Primary Myeloma Cells and Prevents Tumor Growth by Specific Inhibition of Cyclin-Dependent Kinase 4/6. Cancer Research (2006), 66(15), 7661-7667; Fry, D. W. et al. Specific inhibition of cyclin-dependent kinase 4/6 by PD 0332991 and associated antitumor activity in human tumor xenografts. Molecular Cancer Therapeutics, (2004) 3(11), 1427-1438) was used. Palbociclib is a CDK inhibitor, which promotes many markers of cellular senescence (Klein, M. E., et al. CDK4/6 Inhibitors: The Mechanism of Action May Not Be as Simple as Once Thought. Cancer Cell, (2018) 34(1), 9-20*;* Llanos, S., et al. Lysosomal trapping of palbociclib and its functional implications. Oncogene, (2019) 38(20), 3886-3902*),* including an enlarged and flattened cell shape and an enhanced senescence-associated β-galactosidase (SA-β-gal) activity (Figure 1A). Treatment with increasing concentrations of Tvs1 (0.1 nM to 3 µM) led to a dose-dependent reduction in viability of SKMel103 cells (Figure 1B). A therapeutic window delimited by different potencies in proliferative (IC50≈240nM) and palbociclib-treated SKMel103 cells (IC50≈30nM), corresponding to a senolytic index of 8x for Tvs1, was also identified (Figure 4A). The senolysis induced by Tvs1 showed a significant time-dependency even at very low concentrations (10nM, Figure 1C), but it did not impact viability in the control proliferative cells. Moreover, the senolytic efficacy of 10 nM Tvs1 at 96h was independent of the frequency of application (single application 10nM Tvs1; 54±4% vs 10nM Tvs1 every 48h; 53±6%; p=0.9, n=3). Of note, the senolytic potency of Tvs1 is 13 times higher than navitoclax (Figure 1D) with EC50 values of 30nM and 400nM for Tvs1 and navitoclax, respectively. These results demonstrated that Tvs1 induces senolysis in a manner that is concentration and time-dependent, but frequency independent.

Next, it was evaluated the senolytic capacity of Tvs1 in different models of cellular senescence and cancer cell lines. For that purpose, chemotherapy known to induce cellular senescence by genotoxic damage (doxorubicin and bleomycin) or by inhibiting cyclin-dependent kinases (palbociclib) was used. Cellular models of melanoma, lung adenocarcinoma (A549), hepatoma (Huh7) and neuroblastoma (SH-SY5Y) were used. Tvs1G consistently produced a senolytic effect in all the cancerous cell lines tested independently of the stimulus for achieving cellular senescence (Figure 2, Figures 4F, 4G, 4H). The time dependency of the senolytic effect at low concentration was also validated in doxorubicin-induced senescent SKMel-103 treated with 30nM Tvs1 (Figure 3). Additionally, Tvs1 demonstrated a similar potency in all the cancerous senescent cells tested, and its efficacy was similar to the one observed for 1 µM navitoclax.

### Example 2. Molecular modelling

In orderto elucidate whether a specific structural region was responsible for the senolysis of Tvs1, it was performed a molecular modelling to predict and identify consensus recognition motifs within our candidate. Potential recognition motifs for ATM Kinase, Grb2, P85, Aurora A kinase, Nek1/Nek4/Nek9 and common PKC (https://scansite4.mit.edu/#scanProtein) were identified. Accordingly, it was designed and chemically synthesised a series of truncated form that included specific regions for the potential consensus regions identified. Moreover, the effects of Tvs1 were compared with a small protein of the same family which shares a 93% homology (Tvs2), and consequently a very similar 3D structures *(Álvarez et al. 2009;* Pazos, F. et al. Structural and functional characterization of a recombinant sticholysin I (rSt I) from the sea anemone Stichodactyla helianthus. Toxicon, (2006) 48(8), 1083-1094*; Rivera-De-torre et al., 2020).* Despite the homology, the potency of Tvs2 to reduce cell viability in senescent cells was 47 times lower than Tvs1 (Tvs2 IC50≈1.4µM vs Tvs1 IC50≈30nM) with a total lack of differentiation between senescent and proliferative SKMel-103 cells (Figure 3C,4B,4E, SKMel-103 control IC50≈1.3µM vs SKMel-103+palbociclib, IC50≈1.4 µM; SI=0.92). In that regard, the N terminal end of Tvs1 includes a series of acidic residues that contribute to a negative electric charge (E2, D9 and E16) while the net change is neutral in the analogue sequence for Tvs2 (A1, A8, Q15). Moreover, residue D9 in Tvs1 has been suggested to interact through a salt bridge with a distal β-sheet (K68), perhaps allowing folding and stability of the whole structure (Rivera-de-Torre, E., et al. One single salt bridge explains the different cytolytic activities shown by actinoporins sticholysin I and II from the venom of Stichodactyla helianthus. Archives of Biochemistry and Biophysics, (2017), 636, 79-89). Interestingly, substitution of 2 out of the 3 negatively charged residues at the N- terminal end (E2AD9A) reduced the cytotoxicity in both senescent (IC50≈400nM) and proliferative SKMel-103 (IC50≈100nM), narrowing the therapeutic window and lowering the senolytic index to 4x (Figure 4C, 4E). Therefore, negative charges at the N-terminal end play a key role in the senolytic activity Interestingly, Tvs1 E2AD9A maintains certain degree of senolytic index (Figure 4E), with an N-terminal hydropathicity similar to Tvs2 (SI=0.95) and the exact same C-terminal end as Tvs1. This suggested that the polarity and hydrophilicity of the C-terminal might contribute to the senescent selectivity in the cytotoxicity of Tvs1. Next a Met at the N-terminal and 6xHis modification at the C-terminal was included (Tvs1G) that significantly increased the polarity and hydrophilicity of the protein and to assist in immunoassays as well as the purification process. Moreover, a notable differentiation emerged in the predicted polarity between the N-terminal and C-terminal ends when analysing Tvs1, Tvs2 and Tvs1G (Figure 5). This contrast highlights a more hydrophobic N-terminus for Tvs2, whereas Tvs1G showcases a C-terminus with higher hydrophilicity than Tvs1 and Tvs2. This disparity may have implications for structural changes and functional characteristics in these molecules. Surprisingly, the increased polarity in the C-terminus of Tvs1G reduced the cytotoxicity in proliferative cells (IC50≈1µM) but maintained the potency for senolysis (IC50≈32) (Figure 4D). Therefore, the modifications improved the preference of Tvs1G to target senescent cells with an enhanced senolytic index compared to its native form (Sl=8) or navitoclax (SI=22).

The drastic difference in the potency and selectivity of Tvs1 and Tvs2 rely on 12 residues unevenly distributed throughout the entire sequence. The negative charges at the N-terminal confers to Tvs1 and Tvs1G a more polar amino end and a higher senolityc potency than Tvs2 and Tvs1E2AD9A. On the other hand, the higher polarity and positive charges of the carboxyl end in Tvs1G lowered the cytotoxicity toward proliferative cells.

### Example 3. Deregulated pathways.

In this study, it was performed genome-wide gene expression analysis using RNA sequencing to identify the most deregulated genes and pathways after 3, 6 and 12 h of treatment with 100nM Tvs1 in palbociclib-induced senescent cells (Figure 6). The results of this study showed that treatment with Tvs1 consistently deregulated several pathways at 6 and 12 h related to metabolism (pentose phosphate, oxidative phosphorylation, Krebs cycle, metabolism of lipids, aminoacids and nucleotides). The data also showcased activation of DNA mismatch repair and downregulation of key signalling pathways in cancer (TGFβ, JAK STAT, MAPK, NOTCH, PPAR, P53). Furthermore, Tvs1 initiated early deregulation of several sphingolipid biosynthetic and metabolic pathways (sphingomyelin synthase 1-2, sphingomyelin phosphodiesterase 2-4, sphingosine kinase 1, neutral sphingomyelinase activation associated factor and ceramide kinase 1). Sphingolipids are a group of complex lipids that play crucial roles in various cellular processes, including cell growth, differentiation, and apoptosis (Breslow & Weissman, 2010). Therefore, the fact that deregulation in lipid biosynthetic processes appeared only 3 hours after Tvs1 treatment might suggests that these could play a key role in the cellular response to Tvs1 (see lipidomics results, Figure 17). This screening suggested that Tvs1 might hamper not only the viability and metabolism of senescent cells, but perhaps its paracrine function. Particularly, 36 deregulated genes involved in cell crosstalk signalling and 60 deregulated genes involved in cell excitability and ionic homeostasis were identified. The biological processes associated to those deregulated genes (Figure 7A and 7B) are involved in the control of the plasma membrane potential (22 genes, including chloride, calcium-dependent and voltage dependent potassium channels) and the integral calcium homeostasis (17 genes, including store operated calcium modulators, endoplasmic and mitochondrial calcium uniporters) (Figure 7C). Interestingly, it was observed that senescent cells treated with Tvs1G suffer ionic imbalance (Figure 7D).

### Example 4. Mechanism of action. Potassium leak, mitochondrial disfunction and apoptosis.

### Potassium leak

As mentioned above, it has been suggested that function of proteins in model systems can significantly differ to their behavior in natural lipid bilayers. Consequently, in this study, it was investigated electrophysiological changes induced by Tvs1G in proliferative and senescent SKMel-103 cells. Consistent with an effective formation of pores in the membrane of senescent cells, either intracellular diffusion or extracellular superfusion of Tvs1G led to a principal outward current with delayed rectification component (Figure 8A). This current generated by Tvs1G showed a reversal potential around -40mV under physiological concentrations of potassium (5mM:135mM). Importantly, under symmetrical potassium (135mM:135mM), the current displayed a reversal potential close to the expected equilibrium potential for K⁺ (Erev~0 mV). Moreover, senescent SKMel-103 cells treated with Tvs1G 100nM for 15-20h exhibited a hyperpolarized plasma membrane as compared to the senescent SKMel-103 control (Figure 8B). Results demonstrate that Tvs1G effectively form pores that allow leak of K⁺ and plasma membrane hyperpolarization in senescent cells. The observed outward currents in senescent cells might trigger an ionic imbalance through the leak of cytosolic potassium. Recent studies have shown that impairment of the ionic balance by targeting the Na⁺/K⁺ ATPase effectively kills senescent cells *in vitro* and in vivo (*Guerrero et al., 2019; Triana-Martinez et al., 2019).* The senolytic mechanism proposed for the Na⁺/K⁺ ATPase inhibitors (cardiac glycosides in general) rely on sodium accumulation and an indirect increase in the intracellular Ca²⁺ (*Triana-Martinez et al. 2019).* The sustained raise in intracellular Ca²⁺ is a well know initiator of cell death (Orrenius, S., et al. Regulation of cell death: The calcium-apoptosis link. Nature Reviews Molecular Cell Biology, (2003) 4(7), Article 7). Interestingly, it was identified that Tvs1G facilitates the leak of intracellular potassium and the hyperpolarization of the plasma membrane in senescent cells (Figure 8B). This is of importance as membrane hyperpolarization has been shown to reduce the activity of the Na⁺/K⁺ATPase pump (Gadsby, D. C. et al. Voltage dependence of Na/K pump current in isolated heart cells. Nature, (1985) 315(6014), Article 6014*)* and to increase the driving force for the capacitative calcium entry into the cell *(*Funabashi, K. et al. Accelerated Ca2+ entry by membrane hyperpolarization due to Ca2+-activated K+ channel activation in response to histamine in chondrocytes. American Journal of Physiology-Cell Physiology, 2010, 298(4), C786-C797; Lallet-Daher, H. et al. Intermediate-conductance Ca2+-activated K+ channels (IKCa 1) regulate human prostate cancer cell proliferation through a close control of calcium entry. Oncogene, (2009) 28(15), 1792-1806*;* Parekh, A. B. et al. Store-Operated Calcium Channels. Physiological Reviews, (2005) 85(2), 757-810*;* Yang, L., et al. Ca2+ influx and clearance at hyperpolarized membrane potentials modulate spontaneous and stimulated exocytosis in neuroendocrine cells. Cell Calcium, (2020) 87, 102184*;* Yu, X., et al. Calcium influx through hyperpolarization-activated cation channels (Ih channels) contributes to activity-evoked neuronal secretion. Proceedings of the National Academy of Sciences of the United States of America, (2004) 101(4), 1051-1056*).* This is further supported by data demonstrating that the cytotoxicity of Tvs1G in senescent cells is potentiated by digoxin (Tvs1G IC50≈30nM vs Tvs1G+digoxin 0.1 µM) IC50≈4nM) (Figure 8E-G) and high extracellular Ca²⁺ (4mM) (Figure 8C). In addition, it was partially blocked by the Ca²⁺ chelator EGTA (1Mm, Figure 8C) as well as under depolarizing conditions with high extracellular K⁺ (Figure 8D).

### Example 5. Mitochondrial membrane potential, mitochondrial respiration, and apoptosis.

Senescent cells exhibit changes in mitochondrial function, including impaired oxidative phosphorylation and increased production of reactive oxygen species (ROS) (*Chaib, Tchkonia, et al., 2022; Muñoz-Espín* & *Serrano, 2014*)*.* Senolytic compounds have been shown to selectively induce apoptosis in senescent cells, but mediation on ROS production and mitochondrial respiration remain abstract.

First, stress by flow cytometry in proliferative and palbociclib-induced senescent SKMel-103 cells was evaluated using a cell-permeable probe to detect intracellular ROS (H₂DCFDA). Both, proliferative and senescent cells generated ROS (proliferative 16.4±3 % and senescent 13.7 ±2 %) following 24h of treatment with Tvs1 100nM, but not with 100nM of Tvs2 (Figure 9A). However, this change in ROS is not sufficient to explain the observed senolytic effect of Tvs1 given that the proliferative SKMel-103 cells had a higher sensitivity to a cell permeable ROS analogue (tert-butyl-hydroperoxide, figure 9B). To further elucidate on the role of mitochondria, next mitochondrial membrane potential by flow cytometry and metabolic fluxes by Seahorse technology was assessed. Acute application of Tvs1 or Tvs1G produced a transient increased in respiration (within the first 15 minutes) followed by a steady decay in respiration (Figure 10A). This decay in senescent cells (Figure 10B) was similar following the application of Tvs1 (-33.5 ± 7%) and Tvs1G (-31.1 ± 2 %), but significantly reduced in the denatured form of Tvs1G (-9.1± 1 %). On the contrary, the proliferative cells significantly decreased the basal respiration following the acute application of Tvs1G (100nM, denatured Tvs1G -7.7± 2 % vs Tvs1G -15.1± 2 %, n=5-6, p=0.0186). This was translated to half of the response observed in senescent cells (proliferative Tvs1G -15.1± 2 % vs senescent Tvs1G -31.1 ± 2 %, n=5-6, p=0.0003). However, the proliferative cells overcome those changes after 24h (Figure 10D), while senescent cells maintained an impaired respiration. This was characterized by a marked reduction in ATP-coupling, maximal respiration, and spare respiratory capacity with an increase in the proton leak (Figure 10C). Changes in the glycolytic fluxes were not identified (Figure 11). The impaired mitochondrial respiration is consistent with a more depolarized mitochondrial membrane potential in senescent SKMel-103 cells but not in proliferative SKMel-103 cells treated with Tvs1 or Tvs1G (Figure 12), and the increased apoptosis (Figure 13). Similar to the observed changes in respiration, 1h of incubation with 100nM Tvs1G was enough to depolarize the mitochondrial membrane potential of senescent cells (Rhod123, low ΔΨ population, control 11.7± 2 % vs Tvs1G 21.2 ± 3 %, n=3-6, p=0.043).

These effects are consistent with the uncoupled mitochondrial respiration observed with ionophores (Pressman, B. C. Biological Applications of lonophores. Annual Review of Biochemistry, (1976) 45(1), 501-530*;* Shinohara, Y., et al. Permeability transition-independent release of mitochondrial cytochrome c induced by valinomycin. European Journal of Biochemistry, (2002) 269(21), 5224-5230*).* This raises the intriguing question of whether Tvs1G mediates mitochondrial dysfunction by modulating the mitochondrial permeability directly (pore formation) or indirectly through changes in Ca²⁺ (Laskowski, M., et al. What do we not know about mitochondrial potassium channels? Biochimica et Biophysica Acta (BBA) - Bioenergetics, (2016) 1857(8), 1247-1257. https://doi.org/10.1016/j.bbabio.2016.03.007*; Shinohara et al., 2002).* That aside, these alterations in the respiration and mitochondrial membrane potential of senescent cells due to Tvs1G seem irreparable in senescent cells as they appeared within the first hours and were maintained for up to 24h. Importantly, these effects were not recapitulated following the application of vehicle (PBS 0.2%), 100 nM Tvs2 nor 100nM of the denatured from of Tvs1G.

### Example 6. Lipidomics

Recent compilations highlight the significance of specific lipid species in senescence, including their contribution to SASP-associated inflammation (Hamsanathan, S. et al. Lipids as Regulators of Cellular Senescence. Frontiers in Physiology, (2022) 13, 796850*).* While protein regulators of senescence and lipid metabolism are well-studied, the connection between critical regulators of senescence and senescence-associated lipid changes remains unclear. To analyse the changes in the senescent lipidome, we used our standard model of proliferative SKMel-103 and palbociclib-induced senescent SKMel-103 cells. Lipidomics revealed that senescent cells have a distinct lipid profile (Figure 14 and 15) accompanied with an abundance in various lipid classes. Senescent cells consistently exhibited alterations in all functional lipid categories including glycerophospholipids, sphingolipids, storage lipids (Figure 16) and lysolipids (Figure 18A) compared to their proliferative counterparts. For example, senescent cells showed increased levels of phosphatidylcholine (PC), phosphatidylethanolamine ether (PE O-), esters of cholesterol (CE) and triacylglycerol (TAG), while cardiolipin (CL, a mitochondrial lipid), phosphatidylserine (PS) and sphingomyelin (SM) were decreased (Figure 16). Senescent cells presented increases in 4 out of 8 lysolipid identified (Figure 19A), including LPC (lyso-Phosphatidylcholine), LPE-O (lyso-Phosphatidylethanolamine-ether), LPI (lyso-Phosphatidylinositol) and LPS (lyso-Phosphatidylserine). Altogether suggests a re-organization of the plasma senescent membrane and endomembrane system accompanied by a stimulation of lipid storage (Figure 15, 16, 19). Once cells have been exposed to Tvs1G, occurs a senescence-dependent modulation of the lipidomic cellular landscape in lipid storage and lisophospholipids (LPL) cellular content with minimum impact on the cellular content of glycerophospholipids (GL) and sphingolipids (SL) (Figure 17-19). Oppositely to proliferative cells, Tvs1G in senescent cells induced a reduction of CE and TAG lipids that are normally stored in lipid droplets and constitute hallmarks of adiposity and fatty liver, among other lipid disorders. Tvs1G did not alter lysophospholipids concentrations in proliferative cells but in senescent cells diminished lysophosphatidylinositol (LPI) and lysophosphatidylserine (LPS) cellular content, which modulate cell growth and metabolism.

Next, the role of PC, SM and cholesterol in the cytotoxicity and senolysis of Tvs1G was characterized. These lipids are the most abundant within the main functional categories and have been shown to modulate the activity of pore-forming toxins (Palacios-Ortega et al., 2019). In sticholysins particularly, the balance between these three lipids is essential to allow a thermodynamically favourable transition from the hydrophilic form in aqueous medium to the hydrophobic form inserted into the lipid membrane (Garcia-Ortega, L., et al. The behavior of sea anemone actinoporins at the water-membrane interface. Biochimica et Biophysica Acta (BBA) - Biomembranes, (2011) 1808(9), 2275-2288*; Palacios-Ortega et al., 2019).* It was identified that senescent cells have lower levels of SM with a higher content of PC (Figure 15 and 16), consistent with an increased activity in sphingomyelinase (Venable et al., 1995). This led us to hypothesized that the senolytic effect of Tvs1G might rely on a preferential binding to lipids within the plasma membrane of senescent cells. Indeed, PC supplementation (100 µM POPC, 1-palmitoyl-2-oleoylphosphatidylcholine) in proliferative SKMel-103 cells increased cytotoxicity of Tvs1G (Figure 16D), resembling the observed response in senescent cells. Moreover, degradation of SM by exogenous SMAse potentiated the cytotoxicity of Tvs1G in both proliferative and senescent cells. This potentiation, however, could be attributed to increases in the final products of SM degradation (PC+ceramide) or to a decrease in SM content. To decipher SM contribution, the cytotoxic effects of Tvs1G in cells supplemented with 100 µM SM were examined. Interestingly, the addition of SM mitigated the effects of Tvs1G in senescent cells, while potentiating Tvs1G's cytotoxicity in proliferative cells. While the preference of Tvs1G for senescent cells could be well supported by changes in plasma membrane composition (i.e., high PC and low SM content). The cytotoxicity of Tvs1G in proliferative cells highlights a dependence on specific lipid ratios that might mediate membrane fluidity. In that regard, SM has a high affinity for cholesterol and these two lipids pack tightly into liquid-ordered domains among a liquid-disordered phase to form lipid rafts (Lönnfors, M., et al. Sterols Have Higher Affinity for Sphingomyelin than for Phosphatidylcholine Bilayers even at Equal Acyl-Chain Order. Biophysical Journal, (2011) 100(11), 2633-2641*;* Simons, K., et al. Model systems, lipid rafts, and cell membranes. Annual Review of Biophysics and Biomolecular Structure, (2004) 33, 269-295*).* This idea is also supported by the fact that limiting cholesterol availability with lipoprotein depleted serum (LPDS) potentiated the effects of Tvs1G in proliferative cells (Figure 16F), and partially blunted the cytotoxicity in senescent cells. In any case, the principal component analysis revealed that 12h of treatment with Tvs1G is sufficient to remodel the lipid profile in both proliferative and senescent cells (Figure 14), with more profound changes in the former group. This suggests that fluctuations in lipid composition mediates the adaptation in both cell models towards the changes induced by Tvs1G. Administration of Tvs1G increased the levels of major membrane components, such as phosphatidylcholine (PC) and cholesterol (CE, cholesterol esters), in proliferative SKMel-103 cells (Figure 18). However, this effect was not observed in senescent SKMel-103 cells. This suggests that the elevation of PC and CE might be a response aimed at facilitating membrane restoration specifically in proliferative cells. On the other hand, Tvs1G selectively reduced the content of lysophospholypids (LPI, LPS) in senescent cells (Figure 19). These lipids are of particular interest as lysophospholipids are multifunctional mediators in inflammation and paracrine responses (W. M. Chen et al., 2020; Rivera & Chun, 2008) and their reduction might be relevant for SASP modulation by Tvs1G.

### Example 7. Modulation of SASP by Tvs1 and Tvs1G: in vitro.

Senescent cells undergo a process known as senescence-associated secretory phenotype (SASP), which involves the secretion of various factors that contribute to chronic inflammation and age-related diseases (*Chaib, S., et al (2022); Gorgoulis et al., 2019).* The SASP includes inflammatory and pro-apoptotic cytokines (e.g., TNFα, IL-6, IL-8), chemokines, matrix metalloproteinases, TGFβ family members, activins and inhibins, hemostatic and growth factors, bioactive lipids, and exosomes (*Gorgoulis et al., 2019).* These contribute to inflammation and tissue dysfunction and to transfer cytotoxic and senescence-inducing cargos locally and systemically.

In this study, SASP regulation by Tvs1 and its potential as a therapeutic intervention for cellular senescence was investigated, as a key contributor to age-related diseases. To assess the effects of Tvs1 on the senescence-associated secretory phenotype (SASP), cytokine arrays to identify the main SASP components in a cellular model of senescence induced by Palbociclib were performed. Furthermore, SASP factors modulated by Tvs1 were screened. First, changes in SASP induced by Tvs1 in cultures of senescent SKMel-103 cells were evaluated. In brief, palbociclib-induced senescent cells were treated with Tvs1 at a concentration of 10-30 nM for 96 hours. Subsequently, changes in SASP were analysed using a cytokine array that can detect the levels of secretion of cytokines and chemokines. To avoid confusing outcomes derived from cell death, SASP detection was done in 5×10⁵ per group after trypan blue staining to exclude dead cells.

Cellular senescence induced by palbociclib increased several interleukins, chemokines, colony and growth factors (Figure 20) similar to what has already been described (Gorgoulis et al., 2019). Interestingly, some other well-known contributors of SASP were potently induced by palbociclib-induced cellular senescence (IL-6, IL-1β, CCL5, CXCL10, CXCL11, MIP-1a) while others showed mild increases as compared to proliferative cells (IL-1α, CCL2, CXCIL12 and G-SCF). Interestingly, IL-8, serpinE1 and MIF displayed similar expression in senescent and proliferative cells, suggesting that different senescence models may result in distinct patterns of cytokine and chemokine secretion (Özcan et al., 2016). That aside, low concentrations of Tvs1 (10-30nM) were enough to diminish the level of cytokines secretion (IL-6, IL-1P, CCLX10) or fully revert to baseline levels comparable to proliferative cells (IL-1α, CCL2, CCL5, CCL11, MIP-1a, CXCL12 and G-SCF).

SASP modulatory capacity of our candidate TVS1G were further validated in parallel studies targeting skin fibroblast from Hutchinson-Gilford progeria syndrome (HGPS), a rare genetic disorder characterized by accelerated aging in children. It was observed that Tvs1G decreased the viability of senescent HGPS skin fibroblast, accompanied by reductions in β-galactosidase staining. Of note, Tvs1G achieved senolysis in senescent HGPS skin fibroblast, while other senolytics failed (navitoclax, digoxin or dasatinib+quercetin). Moreover, Tvs1G promoted the decrease in 10 cytokines, including interleukins (IL-1α, IL-13, IL-16, IL-17 and IL-18), chemokines (CXCL12, CCL2 and CXCL1), protease inhibitors (Serpin E1) and macrophage migration inhibitory factor (MIF) (Figure 21).

### Example 8. Modulation of SASP by Tvs1G: ex vivo and in vivo in kidneys.

To further validate the potential of our improved candidate to regulate SASP, kidney tissue from 19-22 months old female mice (aged) fed for four months on a high fat diet were treated with Tvs1G ex vivo at a concentration of 100 nM for 48 hours. For these assays, liver, kidney and white adipose tissue were isolated. The tissue was then homogeneously cut into small pieces, weighted, washed with PBS 3 times and cultured in complete DMEM with vehicle or 100 nM of TVS1G. After 48 h, the tissue explants were washed 3 times with PBS and maintained in media without drugs for 24 h to collect the conditioned medium for the mouse cytokine array panels. The volume of media was adjusted according to the weight of the tissue to maintain a ratio of 0.2 g/mL throughout the entire procedure. To analyse the production of cytokines, the conditioned media from 8 independent experiments per tissue were pooled. The tissue explants were then fixed and stained to detect senescence-associated β-galactosidase activity.

Changes in 5 out of the 21 cytokines identified (23.8%) in liver after Tvs1G treatment were observed. Specifically, 3 out of 5 cytokines were increased, including IL-6, C5 and GM-CSF. In the adipose tissue, only 2 chemokines out of the 24 cytokines identified (8.3%) were reduced after ex-vivo treatment with Tvs1G. Interestingly, in the treated kidneys, changes in the release of 20 out of the 21 cytokines (95.2%) were identified. That comprised the reduction of 6 interleukins, including a -86% decrease in the secretion IL-6, and a -63% reduction in the proinflammatory TNFα (Figure 22), known to contribute to tissue damage and chronic kidney disease *(*Lee, B. T., et al. Association of C-reactive protein, tumor necrosis factor-alpha, and interleukin-6 with chronic kidney disease. BMC Nephrology, (2015) 16, 77*;* Navarro, J. F,et al. Renal pro-inflammatory cytokine gene expression in diabetic nephropathy: Effect of angiotensin-converting enzyme inhibition and pentoxifylline administration. American Journal of Nephrology, (2006), 26(6), 562-570*).* These data suggest that TVS1G exerts a deeper impact on the secretory profile of kidneys than liver or adipose tissue.

These data were further validated in vivo in the same model of ageing and inflammation. For that, aged mice (18 months old) fed on a high fat diet for 4 months were injected (i.p) with vehicle (PBS) or Tvs1G 40 µg/Kg every 2-3 days for 1 month. Animals were monitored daily and at the end of the experimentation organs and serum were collected for further analysis. Consistently with the potency of this candidate, a dose as low as 40 µg/Kg was sufficient to induce a -48% reduction in the circulating levels of TNFα in this model.

It was performed senescent associated β-galactosidase staining in kidneys from the *in vivo* and ex vivo studies described above. The percentage of stained area relative were compared to the total area analysed. Four images (10x magnification) were analysed to cover a total of ~4 mm³ per kidney. Staining in kidneys was mainly identified in the convoluted tubules. Analysis revealed an evident reduction in the level of senescence-associated β-galactosidase staining following treatments with Tvs1G in vivo (Figure 23A-B) and ex vivo (Figure 23 C-D)

Altogether, our results showed that Tvs1G treatment significantly reduced the impact of cellular senescence by modulating the levels of several SASP components *in vitro,* ex vivo and in vivo, including pro-inflammatory cytokines tightly related to kidney disease such as TNFα.

### Example 9. Cancer.

Models of xenograft tumours of melanoma SKMel-103 cells in female BALB/c FoxN1 nude mice were generated. Mice were randomly divided in 5 groups of 4 mice each: untreated, palbociclib, Tvs1G, palbociclib + Tvs1G and palbociclib + navitoclax (as a senolytic control).

Tvs1G 2mg/kg reduced tumour progression in xenograft SKMel-103 nude mice models with therapy induced senescence (i.e., palbociclib treatment) and in comparison, to treatment alone (Tvs1G) and control mice cohorts (vehicle- or Palbociclib-treated, Figure 24). Therefore, Tvs1G strongly synergises with a CDK4/6 inhibitor (palbociclib) for remission of solid tumours in mice. In addition, it was found that TVS1G is as effective as navitoclax in the remission of solid tumours in combination with palbociclib (Figure 24).

### Example 10. Lung fibrosis.

To test the hypothesis that Tvs1G ameliorates pulmonary cellular senescence and thus, lung remodelling, the bleomycin fibrosis model to C57BI/6 mice were applied. For that, mice were treated with a single administration of bleomycin (2 U/Kg). After 23 days, Tvs1G (6mg/Kg) was i.p. injected every 2 days for a total of 8 days (4 injections). At the end of the treatments, the modified Ashcroft scale (*Hübner, R.-H., et al (2008*)) was used for the evaluation of the severity of lung injury and fibrosis in stained histological samples by visual assessment. Data revealed that the vehicle treated group presented alveoli thickening variable with confluent fibrotic masses (10%-50% of microscopic field) consistent with a score - 5 of the modified Ashcroft scale. This was perhaps in the lower range of the expected score for the bleomycin model (Chen, L., et al. Blockade of advanced glycation end product formation attenuates bleomycin-induced pulmonary fibrosis in rats. Respiratory Research, (2009) 10(1), 55*;* He, H., et al. Tanshinone IIA attenuates bleomycin-induced pulmonary fibrosis in rats. Molecular Medicine Reports, (2015) 11(6), 4190-4196*;* Li, S., et al. Inhibition of Raf1 ameliorates bleomycin-induced pulmonary fibrosis through attenuation of TGF-β1 signaling. American Journal of Physiology-Lung Cellular and Molecular Physiology, (2018) 315(2), L241-L247*).* That aside, the Tvs1G-treated group presented lesser levels of SA-β-galactosidase staining with significant reductions in the appearance of fibrotic masses (<10% of microscopic field) consistent with lower Ashcroft scores and lung remodelling amelioration (Figure 25).

### Conclusions

Data supports that small proteins of the present invention target senescent cells attending to its binding specificity to lipid membranes. Moreover, it facilitates ionic imbalance with a potassium loss and membrane hyperpolarization in senescent cells. This is accompanied by depolarization of the mitochondrial membrane potential and reduction of mitochondrial ATP-coupling in senescent but not in proliferative cells. The senolytic effect of these proteins was also related to deregulation of 1) genes involved in metabolism and key signalling pathways in cancer, and 2) lipids with structural, metabolic and inflammatory functions.

## Claims

1. An isolated small protein having an amino acid sequence selected from SEQ ID NO 1 or SEQ ID NO 2, or fragments thereof, for use as a senolytic and/or senomorphic agent.

2. The isolated small protein of claim 1 for use in the prevention or treatment of aging and age-related diseases.

3. The isolated small protein for use according to claim 2 wherein the age-related disease is selected from cancer, fibrosis disease, aged-related obesity, kidney disease, and progeria syndrome.

4. The isolated small protein for use according to claim 3 wherein the cancer disease is selected from melanoma, hepatoma, lung adenocarcinoma and neuroblastoma.

5. The isolated small protein for use according to claim 3 or 4 for reducing solid tumour progression.

6. A pharmaceutical or cosmetic composition comprising the small protein according to claims 1-5.

7. The pharmaceutical composition according to claim 6 for use in the prevention or treatment of aging and age-related diseases.

8. The pharmaceutical composition, according to claim 7, optionally comprising a pharmaceutically acceptable excipients or carrier.

9. The pharmaceutical composition according to claim 7 or 8 further comprising at least one further active compound.

10. The pharmaceutical composition according to claim 9 wherein the further active compound is selected from one or more of a CDK4/6 inhibitor, a genotoxic chemotherapy agent and a radioactive compound.

11. The pharmaceutical composition according to claim 9 or 10, further combined with a cardiac glycoside.

12. The cosmetic composition according to claim 6 for use in skin condition improvement.

13. An isolated small protein consisting of SEQ ID NO 2.

14. The isolated small protein according to claim 13 for use as a therapeutic agent.

15. A method for identifying a compound for the treatment or prevention of aging and age-related diseases comprising:
- contacting a senescent cell with a test compound;
- determining if the test compound reduces the lysolipids, LPS and LPI, cellular content;
thereby identifying a compound effective for the treatment or prevention of aging and age-related diseases.
